# EUROPÄISCHE PATENTSCHRIFT

(11) **EP 2 744 896 B1**
(45) Veröffentlichungstag und Bekanntmachung des Hinweises auf die Patenterteilung: **16.03.2016**
(21) Anmeldenummer: 12738448.5
(22) Anmeldetag: 23.07.2012
(51) Int. Cl.: C12N 9/10, C12N 9/88, C12N 9/12, C12N 9/00, C12N 9/04, C07K 14/39, C12P 13/02

(54) **PICHIA CIFERRII ZELLEN UND DEREN VERWENDUNG**
PICHIA CIFERRII CELLS AND USE THEREOF
CELLULES DE PICHIA CIFERRII ET LEUR UTILISATION

(30) Priorität: 18.08.2011 DE 102011110959
(43) Veröffentlichungstag der Anmeldung: 25.06.2014
(73) Patentinhaber: Evonik Degussa GmbH, 45128 Essen (DE)
(72) Erfinder: KÖHLER, Tim, 46284 Dorsten (DE); SCHORSCH, Christoph, 60316 Frankfurt am Main (DE); BOLES, Eckhard, 64293 Darmstadt (DE); ANDREA, Heiko, 45768 Marl (DE); FARWICK, Mike, 45138 Essen (DE); SCHAFFER, Steffen, 45699 Herten (DE)
(86) Internationale Anmeldenummer: PCT/EP2012/064369
(87) Internationale Veröffentlichungsnummer: WO 2013/023878

(56) Entgegenhaltungen:
- WO-A1-95/12683
- WO-A1-2007/131720
- WO-A2-2006/048458
- DATABASE EMBL [Online] 21. Mai 2010 (2010-05-21), "Sequence 10053 from Patent WO2010046221.", XP002683376, gefunden im EBI accession no. EM_PAT:HC752962 Database accession no. HC752962
- DATABASE EMBL [Online] 10. Oktober 2004 (2004-10-10), "Sequence 3361 from patent US 6747137.", XP002683377, gefunden im EBI accession no. EM_PAT:AR548230 Database accession no. AR548230
- DATABASE Geneseq [Online] 1. Juni 2006 (2006-06-01), "C. albicans cell wall protein CHA1-2 DNA SEQ ID NO 176.", XP002683378, gefunden im EBI accession no. GSN:AEG97407 Database accession no. AEG97407
- DATABASE EMBL [Online] 10. Januar 2001 (2001-01-10), "T7 end of clone AT0AA004H05 of library AT0AA from strain CBS 4311 of Saccharomyces servazzii", XP002683379, gefunden im EBI accession no. EM_GSS:AL402816 Database accession no. AL402816
- DATABASE EMBL [Online] 16. April 2009 (2009-04-16), "Sequence 4369 from Patent WO2009037279.", XP002683380, gefunden im EBI accession no. EM_PAT:GN099588 Database accession no. GN099588
- DATABASE EMBL [Online] 8. Juli 2005 (2005-07-08), "cp56b06.r Candida parapsilosis Random Genomic Library Candida parapsilosis genomic clone cp56b06, genomic survey sequence.", XP002683381, gefunden im EBI accession no. EM_GSS:CZ288060 Database accession no. CZ288060
- CHRISTOPH SCHORSCH ET AL: "High-level production of tetraacetyl phytosphingosine (TAPS) by combined genetic engineering of sphingoid base biosynthesis and L-serine availability in the non-conventional yeast Pichia ciferrii", METABOLIC ENGINEERING, Bd. 14, Nr. 2, 1. März 2012 (2012-03-01), Seiten 172-184, XP55038100, ISSN: 1096-7176, DOI: 10.1016/j.ymben.2011.12.002

### SEQUENCE LISTING

<110> Evonik Degussa GmbH
<120> Pichia ciferrii Zellen und deren Verwendung
<130> 201100231
<160> 86
<170> PatentIn version 3.5
<210> 1
   <211> 1347
   <212> DNA
   <213> Pichia ciferrii
<220>
   <221> CDS
   <222> (1)..(1347)
<400> 1
   atg gct gaa atc ctt aaa aat gaa cgt cac aga caa aaa tca tca att      48
   Met Ala Glu Ile Leu Lys Asn Glu Arg His Arg Gln Lys Ser Ser Ile
   1               5                   10                  15
   act tta att cca tca gaa aat ttt aca tca aaa tct gtt atg gat tta      96
   Thr Leu Ile Pro Ser Glu Asn Phe Thr Ser Lys Ser Val Met Asp Leu
               20                  25                  30
   tta ggt tca gaa atg caa aat aaa tat tca gaa ggt tat cca ggt gaa     144
   Leu Gly Ser Glu Met Gln Asn Lys Tyr Ser Glu Gly Tyr Pro Gly Glu
           35                  40                  45
   cgt tat tat ggt ggt aat gaa ttt att gat caa gct gaa gca tta tgt     192
   Arg Tyr Tyr Gly Gly Asn Glu Phe Ile Asp Gln Ala Glu Ala Leu Cys
       50                  55                  60
   caa aaa cgt gct ttg gaa gct ttt aac ttg gat cct gaa tta tgg gga     240
   Gln Lys Arg Ala Leu Glu Ala Phe Asn Leu Asp Pro Glu Leu Trp Gly
   65                  70                  75                  80
   gtt aat gtt caa tct tta tca ggt gca cca gca aat tta tat gct tat     288
   Val Asn Val Gln Ser Leu Ser Gly Ala Pro Ala Asn Leu Tyr Ala Tyr
                   85                  90                  95
   tca tca atc tta aat gtt ggt gat aga att atg ggt ctt gat tta cct     336
   Ser Ser Ile Leu Asn Val Gly Asp Arg Ile Met Gly Leu Asp Leu Pro
               100                 105                 110
   cat ggt ggt cat tta tct cat ggt tat caa act gct aca act aaa atc     384
   His Gly Gly His Leu Ser His Gly Tyr Gln Thr Ala Thr Thr Lys Ile
           115                 120                 125
   tct tat att tca aaa tat ttc caa act atg cca tat aga tta aat gaa     432
   Ser Tyr Ile Ser Lys Tyr Phe Gln Thr Met Pro Tyr Arg Leu Asn Glu
       130                 135                 140
   gaa act ggt ata att gat tat gat gca tta gaa aaa tct gca gaa tta     480
   Glu Thr Gly Ile Ile Asp Tyr Asp Ala Leu Glu Lys Ser Ala Glu Leu
   145                 150                 155                 160
   ttt aga cca aaa atc att gtt gca ggt gct tca gca tat tca aga att         528
   Phe Arg Pro Lys Ile Ile Val Ala Gly Ala Ser Ala Tyr Ser Arg Ile
                   165                 170                 175
   att gat tat gaa aga atc aag aaa atc gca gat aaa gtt aat gct tat         576
   Ile Asp Tyr Glu Arg Ile Lys Lys Ile Ala Asp Lys Val Asn Ala Tyr
               180                 185                 190
   gtg cta tca gat atg gct cat att tca ggt tta gtt tct gca gaa gtt         624
   Val Leu Ser Asp Met Ala His Ile Ser Gly Leu Val Ser Ala Glu Val
           195                 200                 205
   aca cca tca cca ttc cca ttc tca gat att gtt act aca aca act cat         672
   Thr Pro Ser Pro Phe Pro Phe Ser Asp Ile Val Thr Thr Thr Thr His
       210                 215                 220
   aaa tca tta aga ggt cca aga ggt gca atg att ttc ttt aga aaa ggt         720
   Lys Ser Leu Arg Gly Pro Arg Gly Ala Met Ile Phe Phe Arg Lys Gly
   225                 230                 235                 240
   tta aga aaa act act aaa aag ggt aaa gaa att tat tat gat tta gaa         768
   Leu Arg Lys Thr Thr Lys Lys Gly Lys Glu Ile Tyr Tyr Asp Leu Glu
                   245                 250                 255
   aaa aaa att aat ttt tct gtt ttc cca gct cat caa ggt ggt cca cat         816
   Lys Lys Ile Asn Phe Ser Val Phe Pro Ala His Gln Gly Gly Pro His
               260                 265                 270
   aat cat aca att tct gca tta gct gtt gct ttg aaa caa gca caa tct         864
   Asn His Thr Ile Ser Ala Leu Ala Val Ala Leu Lys Gln Ala Gln Ser
           275                 280                 285
   tca gaa tat aaa gaa tat caa caa aat gtt gtt aat aat gca agt cat         912
   Ser Glu Tyr Lys Glu Tyr Gln Gln Asn Val Val Asn Asn Ala Ser His
       290                 295                 300
   ttc gct gat gtt tta caa aca aaa ggt ttt gat tta gtt tct aat ggt         960
   Phe Ala Asp Val Leu Gln Thr Lys Gly Phe Asp Leu Val Ser Asn Gly
   305                 310                 315                 320
   aca gat act cat tta atc ttg att gat tta cgt tcc aaa aaa att gat        1008
   Thr Asp Thr His Leu Ile Leu Ile Asp Leu Arg Ser Lys Lys Ile Asp
                   325                 330                 335
   ggt gca aga tta gaa gct gtt tta gaa aga ata aac att gca gct aat        1056
   Gly Ala Arg Leu Glu Ala Val Leu Glu Arg Ile Asn Ile Ala Ala Asn
               340                 345                 350
   aaa aat act att cca ggt gat aaa tct gct tta ttc cca tca ggt tta        1104
   Lys Asn Thr Ile Pro Gly Asp Lys Ser Ala Leu Phe Pro Ser Gly Leu
           355                 360                 365
   aga gtt ggt act cca gca atg aca aca aga ggt ttt gaa aat aaa gaa        1152
   Arg Val Gly Thr Pro Ala Met Thr Thr Arg Gly Phe Glu Asn Lys Glu
       370                 375                 380
   ttt aat aaa gtt gca gat tat att gat cgt gct gtt aaa tta gct ttg        1200
   Phe Asn Lys Val Ala Asp Tyr Ile Asp Arg Ala Val Lys Leu Ala Leu
   385                 390                 395                 400
   att tta aaa gat caa gct aaa ggt gat gat gca aga gct tta tta gca        1248
   Ile Leu Lys Asp Gln Ala Lys Gly Asp Asp Ala Arg Ala Leu Leu Ala
                   405                 410                 415
   aat ttc aaa aaa tta gct gat gaa tct gat gat gtt aaa gct tta ggt      1296
   Asn Phe Lys Lys Leu Ala Asp Glu Ser Asp Asp Val Lys Ala Leu Gly
               420                 425                 430
   aaa gaa gtt gct gaa tgg gtt tct caa tat cca gtt cca ggt gaa tta      1344
   Lys Glu Val Ala Glu Trp Val Ser Gln Tyr Pro Val Pro Gly Glu Leu
           435                 440                 445
   taa                                                                  1347
<210> 2
   <211> 448
   <212> PRT
   <213> Pichia ciferrii
<400> 2
   Met Ala Glu Ile Leu Lys Asn Glu Arg His Arg Gln Lys Ser Ser Ile
   1               5                   10                  15
   Thr Leu Ile Pro Ser Glu Asn Phe Thr Ser Lys Ser Val Met Asp Leu
               20                  25                  30
   Leu Gly Ser Glu Met Gln Asn Lys Tyr Ser Glu Gly Tyr Pro Gly Glu
           35                  40                  45
   Arg Tyr Tyr Gly Gly Asn Glu Phe Ile Asp Gln Ala Glu Ala Leu Cys
       50                  55                  60
   Gln Lys Arg Ala Leu Glu Ala Phe Asn Leu Asp Pro Glu Leu Trp Gly
   65                  70                  75                  80
   Val Asn Val Gln Ser Leu Ser Gly Ala Pro Ala Asn Leu Tyr Ala Tyr
                   85                  90                  95
   Ser Ser Ile Leu Asn Val Gly Asp Arg Ile Met Gly Leu Asp Leu Pro
               100                 105                 110
   His Gly Gly His Leu Ser His Gly Tyr Gln Thr Ala Thr Thr Lys Ile
           115                 120                 125
   Ser Tyr Ile Ser Lys Tyr Phe Gln Thr Met Pro Tyr Arg Leu Asn Glu
       130                 135                 140
   Glu Thr Gly Ile Ile Asp Tyr Asp Ala Leu Glu Lys Ser Ala Glu Leu
   145                 150                 155                 160
   Phe Arg Pro Lys Ile Ile Val Ala Gly Ala Ser Ala Tyr Ser Arg Ile
                   165                 170                 175
   Ile Asp Tyr Glu Arg Ile Lys Lys Ile Ala Asp Lys Val Asn Ala Tyr
               180                 185                 190
   Val Leu Ser Asp Met Ala His Ile Ser Gly Leu Val Ser Ala Glu Val
           195                 200                 205
   Thr Pro Ser Pro Phe Pro Phe Ser Asp Ile Val Thr Thr Thr Thr His
       210                 215                 220
   Lys Ser Leu Arg Gly Pro Arg Gly Ala Met Ile Phe Phe Arg Lys Gly
   225                 230                 235                 240
   Leu Arg Lys Thr Thr Lys Lys Gly Lys Glu Ile Tyr Tyr Asp Leu Glu
                   245                 250                 255
   Lys Lys Ile Asn Phe Ser Val Phe Pro Ala His Gln Gly Gly Pro His
               260                 265                 270
   Asn His Thr Ile Ser Ala Leu Ala Val Ala Leu Lys Gln Ala Gln Ser
           275                 280                 285
   Ser Glu Tyr Lys Glu Tyr Gln Gln Asn Val Val Asn Asn Ala Ser His
       290                 295                 300
   Phe Ala Asp Val Leu Gln Thr Lys Gly Phe Asp Leu Val Ser Asn Gly
   305                 310                 315                 320
   Thr Asp Thr His Leu Ile Leu Ile Asp Leu Arg Ser Lys Lys Ile Asp
                   325                 330                 335
   Gly Ala Arg Leu Glu Ala Val Leu Glu Arg Ile Asn Ile Ala Ala Asn
               340                 345                 350
   Lys Asn Thr Ile Pro Gly Asp Lys Ser Ala Leu Phe Pro Ser Gly Leu
           355                 360                 365
   Arg Val Gly Thr Pro Ala Met Thr Thr Arg Gly Phe Glu Asn Lys Glu
       370                 375                 380
   Phe Asn Lys Val Ala Asp Tyr Ile Asp Arg Ala Val Lys Leu Ala Leu
   385                 390                 395                 400
   Ile Leu Lys Asp Gln Ala Lys Gly Asp Asp Ala Arg Ala Leu Leu Ala
                   405                 410                 415
   Asn Phe Lys Lys Leu Ala Asp Glu Ser Asp Asp Val Lys Ala Leu Gly
               420                 425                 430
   Lys Glu Val Ala Glu Trp Val Ser Gln Tyr Pro Val Pro Gly Glu Leu
               435                 440                 445
<210> 3
   <211> 1410
   <212> DNA
   <213> Pichia ciferrii
<220>
   <221> CDS
   <222> (1)..(1410)
<400> 3
   atg cca tac gct tta cca gaa tct cac aga caa tta gtc gaa ggt cat       48
   Met Pro Tyr Ala Leu Pro Glu Ser His Arg Gln Leu Val Glu Gly His
   1               5                   10                  15
   tta aaa gat acc gat cca gaa gtt gaa caa atc att aaa gat gaa att       96
   Leu Lys Asp Thr Asp Pro Glu Val Glu Gln Ile Ile Lys Asp Glu Ile
               20                  25                  30
   gaa cgt caa aga cat tca atc gtc tta att gca tca gaa aat ttc act      144
   Glu Arg Gln Arg His Ser Ile Val Leu Ile Ala Ser Glu Asn Phe Thr
           35                  40                  45
   tca act gct gtt ttc gat gct tta gga act cca atg tgt aat aaa tat      192
   Ser Thr Ala Val Phe Asp Ala Leu Gly Thr Pro Met Cys Asn Lys Tyr
       50                  55                  60
   tct gaa ggt tat cca ggt gca aga tat tat ggt ggt aat gaa cat att      240
   Ser Glu Gly Tyr Pro Gly Ala Arg Tyr Tyr Gly Gly Asn Glu His Ile
   65                  70                  75                  80
   gat aga att gaa atc tta tgt caa gaa aga gct tta aaa gct ttt aat      288
   Asp Arg Ile Glu Ile Leu Cys Gln Glu Arg Ala Leu Lys Ala Phe Asn
                   85                  90                  95
   atc act tct gat aaa tgg ggg gtt aat gtt caa act ctt tct ggg tct      336
   Ile Thr Ser Asp Lys Trp Gly Val Asn Val Gln Thr Leu Ser Gly Ser
               100                 105                 110
   cct gct aat tta caa gtt tat caa gct att atg aaa cct cat gaa aga      384
   Pro Ala Asn Leu Gln Val Tyr Gln Ala Ile Met Lys Pro His Glu Arg
           115                 120                 125
   tta atg ggt ctt gat tta cct cat ggt ggt cat tta tct cat ggt tat      432
   Leu Met Gly Leu Asp Leu Pro His Gly Gly His Leu Ser His Gly Tyr
       130                 135                 140
   caa act gat act aga aaa atc tct gct gtt tca act tat ttt gaa act      480
   Gln Thr Asp Thr Arg Lys Ile Ser Ala Val Ser Thr Tyr Phe Glu Thr
   145                 150                 155                 160
   atg cct tat aga gtt gat tta gaa act ggt att att gat tat gat acc      528
   Met Pro Tyr Arg Val Asp Leu Glu Thr Gly Ile Ile Asp Tyr Asp Thr
                   165                 170                 175
   tta gaa aag aat gcc tta tta ttc aga cct aag gtc ctt gtt gct ggt      576
   Leu Glu Lys Asn Ala Leu Leu Phe Arg Pro Lys Val Leu Val Ala Gly
               180                 185                 190
   act tct gct tat tgt aga tta att gat tat aaa aga atg aga gaa att      624
   Thr Ser Ala Tyr Cys Arg Leu Ile Asp Tyr Lys Arg Met Arg Glu Ile
           195                 200                 205
   gct gat aaa gtt ggt gct tat tta gtt gtt gat atg gct cat att tca      672
   Ala Asp Lys Val Gly Ala Tyr Leu Val Val Asp Met Ala His Ile Ser
       210                 215                 220
   ggt tta atc gct gct ggt gtt atc cca tct cca ttt gaa tat gct gat      720
   Gly Leu Ile Ala Ala Gly Val Ile Pro Ser Pro Phe Glu Tyr Ala Asp
   225                 230                 235                 240
   att gtc act aca act aca cat aaa tcc cta aga ggt cca aga ggt gcc      768
   Ile Val Thr Thr Thr Thr His Lys Ser Leu Arg Gly Pro Arg Gly Ala
                   245                 250                 255
   atg att ttc ttt aga aga ggt gtt aga tca att aac gct aaa act ggt      816
   Met Ile Phe Phe Arg Arg Gly Val Arg Ser Ile Asn Ala Lys Thr Gly
               260                 265                 270
   gct gaa att aaa tat gat tta gaa aat cca att aat ttc tca gtt ttc      864
   Ala Glu Ile Lys Tyr Asp Leu Glu Asn Pro Ile Asn Phe Ser Val Phe
           275                 280                 285
   cca ggt cat caa ggt ggt cca cat aat cat act att acc gcg tta gca      912
   Pro Gly His Gln Gly Gly Pro His Asn His Thr Ile Thr Ala Leu Ala
       290                 295                 300
   aca gca tta aaa caa gct tca act cca gaa ttt aaa caa tat caa gaa      960
   Thr Ala Leu Lys Gln Ala Ser Thr Pro Glu Phe Lys Gln Tyr Gln Glu
   305                 310                 315                 320
   caa gtt tta aaa aat gct aaa gct tta gaa gaa gaa ttc tta aaa tta     1008
   Gln Val Leu Lys Asn Ala Lys Ala Leu Glu Glu Glu Phe Leu Lys Leu
                   325                 330                 335
   tct tat aaa tta gtt tca aat ggt act gat tct cat atg gtt tta gtt     1056
   Ser Tyr Lys Leu Val Ser Asn Gly Thr Asp Ser His Met Val Leu Val
               340                 345                 350
   tca tta aaa gat aaa ggt atc gat ggt gca aga att gaa acc gtt tgt     1104
   Ser Leu Lys Asp Lys Gly Ile Asp Gly Ala Arg Ile Glu Thr Val Cys
           355                 360                 365
   gaa aac ata aac att gcc tta aac aaa aac tca atc cca ggt gat aaa     1152
   Glu Asn Ile Asn Ile Ala Leu Asn Lys Asn Ser Ile Pro Gly Asp Lys
       370                 375                 380
   tcc gct ctt gtg cca ggt ggt att aga att ggt gca cca gca atg tct     1200
   Ser Ala Leu Val Pro Gly Gly Ile Arg Ile Gly Ala Pro Ala Met Ser
   385                 390                 395                 400
   aca aga ggt ctt ggt gaa gaa gat ttt aaa aaa att gca cat tat att     1248
   Thr Arg Gly Leu Gly Glu Glu Asp Phe Lys Lys Ile Ala His Tyr Ile
                   405                 410                 415
   gat tgg tct gtt caa tat gct aaa aaa att caa agt gaa tta cca aaa     1296
   Asp Trp Ser Val Gln Tyr Ala Lys Lys Ile Gln Ser Glu Leu Pro Lys
               420                 425                 430
   gaa gct aat aga tta aaa gat ttt aaa gct aag att gct caa ggt tct     1344
   Glu Ala Asn Arg Leu Lys Asp Phe Lys Ala Lys Ile Ala Gln Gly Ser
           435                 440                 445
   gat gaa tta act aaa act aag aat gaa att tat gaa tgg gct ggt gaa       1392
   Asp Glu Leu Thr Lys Thr Lys Asn Glu Ile Tyr Glu Trp Ala Gly Glu
       450                 455                 460
   ttc cca tta tct gtt taa                                               1410
   Phe Pro Leu Ser Val
   465
<210> 4
   <211> 469
   <212> PRT
   <213> Pichia ciferrii
<400> 4
   Met Pro Tyr Ala Leu Pro Glu Ser His Arg Gln Leu Val Glu Gly His
   1               5                   10                  15
   Leu Lys Asp Thr Asp Pro Glu Val Glu Gln Ile Ile Lys Asp Glu Ile
               20                  25                  30
   Glu Arg Gln Arg His Ser Ile Val Leu Ile Ala Ser Glu Asn Phe Thr
           35                  40                  45
   Ser Thr Ala Val Phe Asp Ala Leu Gly Thr Pro Met Cys Asn Lys Tyr
       50                  55                  60
   Ser Glu Gly Tyr Pro Gly Ala Arg Tyr Tyr Gly Gly Asn Glu His Ile
   65                  70                  75                  80
   Asp Arg Ile Glu Ile Leu Cys Gln Glu Arg Ala Leu Lys Ala Phe Asn
                   85                  90                  95
   Ile Thr Ser Asp Lys Trp Gly Val Asn Val Gln Thr Leu Ser Gly Ser
               100                 105                 110
   Pro Ala Asn Leu Gln Val Tyr Gln Ala Ile Met Lys Pro His Glu Arg
           115                 120                 125
   Leu Met Gly Leu Asp Leu Pro His Gly Gly His Leu Ser His Gly Tyr
       130                 135                 140
   Gln Thr Asp Thr Arg Lys Ile Ser Ala Val Ser Thr Tyr Phe Glu Thr
   145                 150                 155                 160
   Met Pro Tyr Arg Val Asp Leu Glu Thr Gly Ile Ile Asp Tyr Asp Thr
                   165                 170                 175
   Leu Glu Lys Asn Ala Leu Leu Phe Arg Pro Lys Val Leu Val Ala Gly
   180 185 190
   Thr Ser Ala Tyr Cys Arg Leu Ile Asp Tyr Lys Arg Met Arg Glu Ile
   195 200 205
   Ala Asp Lys Val Gly Ala Tyr Leu Val Val Asp Met Ala His Ile Ser
   210 215 220
   Gly Leu Ile Ala Ala Gly Val Ile Pro Ser Pro Phe Glu Tyr Ala Asp
   225 230 235 240
   Ile Val Thr Thr Thr Thr His Lys Ser Leu Arg Gly Pro Arg Gly Ala
   245 250 255
   Met Ile Phe Phe Arg Arg Gly Val Arg Ser Ile Asn Ala Lys Thr Gly
   260 265 270
   Ala Glu Ile Lys Tyr Asp Leu Glu Asn Pro Ile Asn Phe Ser Val Phe
   275 280 285
   Pro Gly His Gln Gly Gly Pro His Asn His Thr Ile Thr Ala Leu Ala
   290 295 300
   Thr Ala Leu Lys Gln Ala Ser Thr Pro Glu Phe Lys Gln Tyr Gln Glu
   305 310 315 320
   Gln Val Leu Lys Asn Ala Lys Ala Leu Glu Glu Glu Phe Leu Lys Leu
   325 330 335
   Ser Tyr Lys Leu Val Ser Asn Gly Thr Asp Ser His Met Val Leu Val
   340 345 350
   Ser Leu Lys Asp Lys Gly Ile Asp Gly Ala Arg Ile Glu Thr Val Cys
   355 360 365
   Glu Asn Ile Asn Ile Ala Leu Asn Lys Asn Ser Ile Pro Gly Asp Lys
   370 375 380
   Ser Ala Leu Val Pro Gly Gly Ile Arg Ile Gly Ala Pro Ala Met Ser
   385 390 395 400
   Thr Arg Gly Leu Gly Glu Glu Asp Phe Lys Lys Ile Ala His Tyr Ile
   405 410 415
   Asp Trp Ser Val Gln Tyr Ala Lys Lys Ile Gln Ser Glu Leu Pro Lys
   420 425 430
   Glu Ala Asn Arg Leu Lys Asp Phe Lys Ala Lys Ile Ala Gln Gly Ser
           435                 440                 445
   Asp Glu Leu Thr Lys Thr Lys Asn Glu Ile Tyr Glu Trp Ala Gly Glu
       450                 455                 460
   Phe Pro Leu Ser Val
   465
<210> 5
   <211> 1029
   <212> DNA
   <213> Pichia ciferrii
<220>
   <221> CDS
   <222> (1)..(1029)
<400> 5
   atg aca atc aca aaa gat cat aaa gtc cca tac atc aag act cca tta        48
   Met Thr Ile Thr Lys Asp His Lys Val Pro Tyr Ile Lys Thr Pro Leu
   1               5                   10                  15
   gtt gat tgt aaa gaa cta tca gaa caa tca cca tgt aga ata ttc cta        96
   Val Asp Cys Lys Glu Leu Ser Glu Gln Ser Pro Cys Arg Ile Phe Leu
               20                  25                  30
   aag caa gaa ttc att caa cca tcg ggt tct tac aaa ata cgt gga ctt       144
   Lys Gln Glu Phe Ile Gln Pro Ser Gly Ser Tyr Lys Ile Arg Gly Leu
           35                  40                  45
   tca aat tta att aga act tca att gaa gaa att aaa tca aat cct aat       192
   Ser Asn Leu Ile Arg Thr Ser Ile Glu Glu Ile Lys Ser Asn Pro Asn
       50                  55                  60
   aat ttg ggt aaa aca att cat gtt tat gct gct tct ggt ggt aat gct       240
   Asn Leu Gly Lys Thr Ile His Val Tyr Ala Ala Ser Gly Gly Asn Ala
   65                  70                  75                  80
   ggt aat gct gtc tct tgt gct tct caa ttt tat gga tta gaa tca aca       288
   Gly Asn Ala Val Ser Cys Ala Ser Gln Phe Tyr Gly Leu Glu Ser Thr
                   85                  90                  95
   gtt gtt ata cca aaa gct aca agt gat aaa atg aag caa aaa atc ttt       336
   Val Val Ile Pro Lys Ala Thr Ser Asp Lys Met Lys Gln Lys Ile Phe
               100                 105                 110
   aaa aat gga tca aaa ata att gtt caa ggt gaa act att ggt gaa gct       384
   Lys Asn Gly Ser Lys Ile Ile Val Gln Gly Glu Thr Ile Gly Glu Ala
           115                 120                 125
   gca att tat tta aaa gat gtc tta atc cct tca tta gat gat tct att       432
   Ala Ile Tyr Leu Lys Asp Val Leu Ile Pro Ser Leu Asp Asp Ser Ile
       130                 135                 140
   ata cct atc tat tgt cat cct tat gat atc cca gct ata tgg cat ggt       480
   Ile Pro Ile Tyr Cys His Pro Tyr Asp Ile Pro Ala Ile Trp His Gly
   145                 150                 155                 160
   cat tct tct att ata gat gaa att gtt gat caa ttg gcc tct tca aat         528
   His Ser Ser Ile Ile Asp Glu Ile Val Asp Gln Leu Ala Ser Ser Asn
                   165                 170                 175
   gaa tta tca aaa tta aaa ggt att gtt tgt tca att ggt ggt ggt gga         576
   Glu Leu Ser Lys Leu Lys Gly Ile Val Cys Ser Ile Gly Gly Gly Gly
               180                 185                 190
   ctt tat aat ggt tta gtt caa ggt tta caa aga aat caa tta tca aaa         624
   Leu Tyr Asn Gly Leu Val Gln Gly Leu Gln Arg Asn Gln Leu Ser Lys
           195                 200                 205
   att cca ata atg act tta gaa aca gat act tgt cca act ttc cat gaa         672
   Ile Pro Ile Met Thr Leu Glu Thr Asp Thr Cys Pro Thr Phe His Glu
       210                 215                 220
   tct att aaa gca caa aaa caa gta ttc att aaa aaa acc aat aca att         720
   Ser Ile Lys Ala Gln Lys Gln Val Phe Ile Lys Lys Thr Asn Thr Ile
   225                 230                 235                 240
   gca att tct tta gct tgt cct tat gtc tct ttg aaa act ctt gaa tat         768
   Ala Ile Ser Leu Ala Cys Pro Tyr Val Ser Leu Lys Thr Leu Glu Tyr
                   245                 250                 255
   tat aat tct cac aag act aag aat tta tta gtt agt gat tct gat gct         816
   Tyr Asn Ser His Lys Thr Lys Asn Leu Leu Val Ser Asp Ser Asp Ala
               260                 265                 270
   gca aat tct tgt tta aat ttt gca aat gaa ttt aat att ata gtg gaa         864
   Ala Asn Ser Cys Leu Asn Phe Ala Asn Glu Phe Asn Ile Ile Val Glu
           275                 280                 285
   cct gct tgt gga gtt gct ttg tgc agt gtt tat aat aat ttg att caa         912
   Pro Ala Cys Gly Val Ala Leu Cys Ser Val Tyr Asn Asn Leu Ile Gln
       290                 295                 300
   aaa aat att gaa ttt ttt gat gat tta aaa tct gat gat att gtg gtt         960
   Lys Asn Ile Glu Phe Phe Asp Asp Leu Lys Ser Asp Asp Ile Val Val
   305                 310                 315                 320
   att att gtt tgt ggt ggg agt tca aca acc gtt caa gat tta aca aat        1008
   Ile Ile Val Cys Gly Gly Ser Ser Thr Thr Val Gln Asp Leu Thr Asn
                   325                 330                 335
   tat aaa cta ctc tat cat tag                                            1029
   Tyr Lys Leu Leu Tyr His
               340
<210> 6
   <211> 342
   <212> PRT
   <213> Pichia ciferrii
<400> 6
   Met Thr Ile Thr Lys Asp His Lys Val Pro Tyr Ile Lys Thr Pro Leu
   1               5                   10                  15
   Val Asp Cys Lys Glu Leu Ser Glu Gln Ser Pro Cys Arg Ile Phe Leu
               20                  25                  30
   Lys Gln Glu Phe Ile Gln Pro Ser Gly Ser Tyr Lys Ile Arg Gly Leu
           35                  40                  45
   Ser Asn Leu Ile Arg Thr Ser Ile Glu Glu Ile Lys Ser Asn Pro Asn
       50                  55                  60
   Asn Leu Gly Lys Thr Ile His Val Tyr Ala Ala Ser Gly Gly Asn Ala
   65                  70                  75                  80
   Gly Asn Ala Val Ser Cys Ala Ser Gln Phe Tyr Gly Leu Glu Ser Thr
                   85                  90                  95
   Val Val Ile Pro Lys Ala Thr Ser Asp Lys Met Lys Gln Lys Ile Phe
               100                 105                 110
   Lys Asn Gly Ser Lys Ile Ile Val Gln Gly Glu Thr Ile Gly Glu Ala
           115                 120                 125
   Ala Ile Tyr Leu Lys Asp Val Leu Ile Pro Ser Leu Asp Asp Ser Ile
       130                 135                 140
   Ile Pro Ile Tyr Cys His Pro Tyr Asp Ile Pro Ala Ile Trp His Gly
   145                 150                 155                 160
   His Ser Ser Ile Ile Asp Glu Ile Val Asp Gln Leu Ala Ser Ser Asn
                   165                 170                 175
   Glu Leu Ser Lys Leu Lys Gly Ile Val Cys Ser Ile Gly Gly Gly Gly
               180                 185                 190
   Leu Tyr Asn Gly Leu Val Gln Gly Leu Gln Arg Asn Gln Leu Ser Lys
           195                 200                 205
   Ile Pro Ile Met Thr Leu Glu Thr Asp Thr Cys Pro Thr Phe His Glu
       210                 215                 220
   Ser Ile Lys Ala Gln Lys Gln Val Phe Ile Lys Lys Thr Asn Thr Ile
   225                 230                 235                 240
   Ala Ile Ser Leu Ala Cys Pro Tyr Val Ser Leu Lys Thr Leu Glu Tyr
                   245                 250                 255
   Tyr Asn Ser His Lys Thr Lys Asn Leu Leu Val Ser Asp Ser Asp Ala
               260                 265                 270
   Ala Asn Ser Cys Leu Asn Phe Ala Asn Glu Phe Asn Ile Ile Val Glu
           275                 280                 285
   Pro Ala Cys Gly Val Ala Leu Cys Ser Val Tyr Asn Asn Leu Ile Gln
       290                 295                 300
   Lys Asn Ile Glu Phe Phe Asp Asp Leu Lys Ser Asp Asp Ile Val Val
   305                 310                 315                 320
   Ile Ile Val Cys Gly Gly Ser Ser Thr Thr Val Gln Asp Leu Thr Asn
                   325                 330                 335
   Tyr Lys Leu Leu Tyr His
               340
<210> 7
   <211> 1530
   <212> DNA
   <213> Pichia ciferrii
<220>
   <221> CDS
   <222> (1)..(1530)
<400> 7
   atg ccg agt ttt gac tct caa aga att aaa ctg atg gat aca gta tca        48
   Met Pro Ser Phe Asp Ser Gln Arg Ile Lys Leu Met Asp Thr Val Ser
   1               5                   10                  15
   gta aac ccc cca aga gcc atc att ggt gat aca ggt atc atc att aaa        96
   Val Asn Pro Pro Arg Ala Ile Ile Gly Asp Thr Gly Ile Ile Ile Lys
               20                  25                  30
   gat caa tct tca ttc tat tac aat caa cat gat aac gct tca ttt tca       144
   Asp Gln Ser Ser Phe Tyr Tyr Asn Gln His Asp Asn Ala Ser Phe Ser
           35                  40                  45
   tct tgc tta agt tgc tct tca tca aac tcg aat ggt acg gtg aaa tct       192
   Ser Cys Leu Ser Cys Ser Ser Ser Asn Ser Asn Gly Thr Val Lys Ser
       50                  55                  60
   tca ggt cca aaa cat att cca ttt gtt gat ata ttg agt gtt cga tat       240
   Ser Gly Pro Lys His Ile Pro Phe Val Asp Ile Leu Ser Val Arg Tyr
   65                  70                  75                  80
   ata aat gaa aat aat gaa tct tta tta gaa gct gga agt tcc act gtg       288
   Ile Asn Glu Asn Asn Glu Ser Leu Leu Glu Ala Gly Ser Ser Thr Val
                   85                  90                  95
   act agt gat gaa cct gat gtc gaa gtg gta ttt gtt aga caa aag ggt       336
   Thr Ser Asp Glu Pro Asp Val Glu Val Val Phe Val Arg Gln Lys Gly
               100                 105                 110
   aaa act ctt gta cca act cca ata ata tta tca ata gat act tta ggt       384
   Lys Thr Leu Val Pro Thr Pro Ile Ile Leu Ser Ile Asp Thr Leu Gly
           115                 120                 125
   cat gat gat gtt gta caa gaa att tgg aga tta agt tat caa gga aca       432
   His Asp Asp Val Val Gln Glu Ile Trp Arg Leu Ser Tyr Gln Gly Thr
       130                 135                 140
   aaa cca aga aaa tca ata tta gtt ctt gtt aat cca cat ggt ggg aaa          480
   Lys Pro Arg Lys Ser Ile Leu Val Leu Val Asn Pro His Gly Gly Lys
   145                 150                 155                 160
   ggt aaa gct ata aat tca ttc tta act caa tca aaa cct gta tta att          528
   Gly Lys Ala Ile Asn Ser Phe Leu Thr Gln Ser Lys Pro Val Leu Ile
                   165                 170                 175
   ggt gct caa gct tct gtt gaa gtt aga cat act caa tat tat caa cat          576
   Gly Ala Gln Ala Ser Val Glu Val Arg His Thr Gln Tyr Tyr Gln His
               180                 185                 190
   gct aca gat att gca cgc act ttg aat att gat aaa tat gat ata att          624
   Ala Thr Asp Ile Ala Arg Thr Leu Asn Ile Asp Lys Tyr Asp Ile Ile
           195                 200                 205
   gca tgt gct tca ggt gat ggt gtc cca cat gaa gtc ttg aat gga ttt          672
   Ala Cys Ala Ser Gly Asp Gly Val Pro His Glu Val Leu Asn Gly Phe
       210                 215                 220
   tat caa aga tct gat aga gct gaa gct ttc aat aag att aca ata act          720
   Tyr Gln Arg Ser Asp Arg Ala Glu Ala Phe Asn Lys Ile Thr Ile Thr
   225                 230                 235                 240
   caa tta cca tgt ggt tca ggt aat gca atg agt gaa tca tgt cat ggt          768
   Gln Leu Pro Cys Gly Ser Gly Asn Ala Met Ser Glu Ser Cys His Gly
                   245                 250                 255
   aca aat aat cca agt ttt gcc gct cta tca tta ttg aaa tca agt acg          816
   Thr Asn Asn Pro Ser Phe Ala Ala Leu Ser Leu Leu Lys Ser Ser Thr
               260                 265                 270
   gta aat tta gat tta atg gct tgt aca caa ggt gat aaa act tat gtt          864
   Val Asn Leu Asp Leu Met Ala Cys Thr Gln Gly Asp Lys Thr Tyr Val
           275                 280                 285
   tca ttc tta agt caa act gtc ggt gtt ata gca gat tct gat att ggt          912
   Ser Phe Leu Ser Gln Thr Val Gly Val Ile Ala Asp Ser Asp Ile Gly
       290                 295                 300
   act gaa gca ctt aga tgg tta ggt cct tca aga ttt gaa tta ggt gtt          960
   Thr Glu Ala Leu Arg Trp Leu Gly Pro Ser Arg Phe Glu Leu Gly Val
   305                 310                 315                 320
   gct tat aaa gtt tta tca aga tca aga tat cca tgt gat ata tct gtt         1008
   Ala Tyr Lys Val Leu Ser Arg Ser Arg Tyr Pro Cys Asp Ile Ser Val
                   325                 330                 335
   aaa tat gct gca aaa tcg aaa aat gaa tta aga caa cat ttt gat gaa         1056
   Lys Tyr Ala Ala Lys Ser Lys Asn Glu Leu Arg Gln His Phe Asp Glu
               340                 345                 350
   cat tcc act att gtt tca aca aaa gat atc caa ata act gaa gat act         1104
   His Ser Thr Ile Val Ser Thr Lys Asp Ile Gln Ile Thr Glu Asp Thr
           355                 360                 365
   tat aat tta aaa tat gat cca aat ggt cca ata cct gat gat tgg gaa         1152
   Tyr Asn Leu Lys Tyr Asp Pro Asn Gly Pro Ile Pro Asp Asp Trp Glu
       370                 375                 380
   gag att gat aaa gat ctt tca gaa aat tta ggt att ttc tat aca ggt         1200
   Glu Ile Asp Lys Asp Leu Ser Glu Asn Leu Gly Ile Phe Tyr Thr Gly
   385                 390                 395                 400
   aaa atg cca tat att gca aaa gat gtt caa ttt ttc cct gca gct tta       1248
   Lys Met Pro Tyr Ile Ala Lys Asp Val Gln Phe Phe Pro Ala Ala Leu
                   405                 410                 415
   cca aat gat ggt act ttt gat tta gtt ata aca gat gct cgt aca agt       1296
   Pro Asn Asp Gly Thr Phe Asp Leu Val Ile Thr Asp Ala Arg Thr Ser
               420                 425                 430
   ata gca cgt atg gca cca act tta tta tca tta gat caa ggt tct cat       1344
   Ile Ala Arg Met Ala Pro Thr Leu Leu Ser Leu Asp Gln Gly Ser His
           435                 440                 445
   gtt tta caa cca gaa gtt caa cat tct aaa ata ata gca tat aga tta       1392
   Val Leu Gln Pro Glu Val Gln His Ser Lys Ile Ile Ala Tyr Arg Leu
       450                 455                 460
   act cca aag cag caa cat ggt tat tta agt gtt gat ggt gaa agt tat       1440
   Thr Pro Lys Gln Gln His Gly Tyr Leu Ser Val Asp Gly Glu Ser Tyr
   465                 470                 475                 480
   cca ttt gaa act att caa gtt gaa att cta ccc ggt gct gca aag act       1488
   Pro Phe Glu Thr Ile Gln Val Glu Ile Leu Pro Gly Ala Ala Lys Thr
                   485                 490                 495
   tta cta aga aat ggt act tat gtt gaa aca aat ttt tat tga               1530
   Leu Leu Arg Asn Gly Thr Tyr Val Glu Thr Asn Phe Tyr
               500                 505
<210> 8
   <211> 509
   <212> PRT
   <213> Pichia ciferrii
<400> 8
   Met Pro Ser Phe Asp Ser Gln Arg Ile Lys Leu Met Asp Thr Val Ser
   1               5                   10                  15
   Val Asn Pro Pro Arg Ala Ile Ile Gly Asp Thr Gly Ile Ile Ile Lys
               20                  25                  30
   Asp Gln Ser Ser Phe Tyr Tyr Asn Gln His Asp Asn Ala Ser Phe Ser
           35                  40                  45
   Ser Cys Leu Ser Cys Ser Ser Ser Asn Ser Asn Gly Thr Val Lys Ser
       50                  55                  60
   Ser Gly Pro Lys His Ile Pro Phe Val Asp Ile Leu Ser Val Arg Tyr
   65                  70                  75                  80
   Ile Asn Glu Asn Asn Glu Ser Leu Leu Glu Ala Gly Ser Ser Thr Val
                   85                  90                  95
   Thr Ser Asp Glu Pro Asp Val Glu Val Val Phe Val Arg Gln Lys Gly
               100                 105                 110
   Lys Thr Leu Val Pro Thr Pro Ile Ile Leu Ser Ile Asp Thr Leu Gly
           115                 120                 125
   His Asp Asp Val Val Gln Glu Ile Trp Arg Leu Ser Tyr Gln Gly Thr
       130                 135                 140
   Lys Pro Arg Lys Ser Ile Leu Val Leu Val Asn Pro His Gly Gly Lys
   145                 150                 155                 160
   Gly Lys Ala Ile Asn Ser Phe Leu Thr Gln Ser Lys Pro Val Leu Ile
                   165                 170                 175
   Gly Ala Gln Ala Ser Val Glu Val Arg His Thr Gln Tyr Tyr Gln His
               180                 185                 190
   Ala Thr Asp Ile Ala Arg Thr Leu Asn Ile Asp Lys Tyr Asp Ile Ile
           195                 200                 205
   Ala Cys Ala Ser Gly Asp Gly Val Pro His Glu Val Leu Asn Gly Phe
       210                 215                 220
   Tyr Gln Arg Ser Asp Arg Ala Glu Ala Phe Asn Lys Ile Thr Ile Thr
   225                 230                 235                 240
   Gln Leu Pro Cys Gly Ser Gly Asn Ala Met Ser Glu Ser Cys His Gly
                   245                 250                 255
   Thr Asn Asn Pro Ser Phe Ala Ala Leu Ser Leu Leu Lys Ser Ser Thr
               260                 265                 270
   Val Asn Leu Asp Leu Met Ala Cys Thr Gln Gly Asp Lys Thr Tyr Val
           275                 280                 285
   Ser Phe Leu Ser Gln Thr Val Gly Val Ile Ala Asp Ser Asp Ile Gly
       290                 295                 300
   Thr Glu Ala Leu Arg Trp Leu Gly Pro Ser Arg Phe Glu Leu Gly Val
   305                 310                 315                 320
   Ala Tyr Lys Val Leu Ser Arg Ser Arg Tyr Pro Cys Asp Ile Ser Val
                   325                 330                 335
   Lys Tyr Ala Ala Lys Ser Lys Asn Glu Leu Arg Gln His Phe Asp Glu
               340                 345                 350
   His Ser Thr Ile Val Ser Thr Lys Asp Ile Gln Ile Thr Glu Asp Thr
   355 360 365
   Tyr Asn Leu Lys Tyr Asp Pro Asn Gly Pro Ile Pro Asp Asp Trp Glu
   370 375 380
   Glu Ile Asp Lys Asp Leu Ser Glu Asn Leu Gly Ile Phe Tyr Thr Gly
   385 390 395 400
   Lys Met Pro Tyr Ile Ala Lys Asp Val Gln Phe Phe Pro Ala Ala Leu
   405 410 415
   Pro Asn Asp Gly Thr Phe Asp Leu Val Ile Thr Asp Ala Arg Thr Ser
   420 425 430
   Ile Ala Arg Met Ala Pro Thr Leu Leu Ser Leu Asp Gln Gly Ser His
   435 440 445
   Val Leu Gln Pro Glu Val Gln His Ser Lys Ile Ile Ala Tyr Arg Leu
   450 455 460
   Thr Pro Lys Gln Gln His Gly Tyr Leu Ser Val Asp Gly Glu Ser Tyr
   465 470 475 480
   Pro Phe Glu Thr Ile Gln Val Glu Ile Leu Pro Gly Ala Ala Lys Thr
   485 490 495
   Leu Leu Arg Asn Gly Thr Tyr Val Glu Thr Asn Phe Tyr
   500 505
<210> 9
   <211> 1515
   <212> DNA
   <213> Pichia ciferrii
<220>
   <221> CDS
   <222> (1)..(1515)
<400> 9
   ttg gcg gtt aac atc act ggt tat ggg tta atc ggc tac tta aag atc      48
   Met Ala Val Asn Ile Thr Gly Tyr Gly Leu Ile Gly Tyr Leu Lys Ile
   1               5                   10                  15
   gta tat aat gaa tta gca aaa gct gta ttc aga aca ttt tta tcc tta      96
   Val Tyr Asn Glu Leu Ala Lys Ala Val Phe Arg Thr Phe Leu Ser Leu
               20                  25                  30
   cca ttt gtt aaa agt aaa gtt gat tca gaa gtt aga gaa aat ttg gac     144
   Pro Phe Val Lys Ser Lys Val Asp Ser Glu Val Arg Glu Asn Leu Asp
           35                  40                  45
   aaa tta gaa gat tct tta att gtc aaa aca cca aat gtt caa gat ttc        192
   Lys Leu Glu Asp Ser Leu Ile Val Lys Thr Pro Asn Val Gln Asp Phe
       50                  55                  60
   caa tca ata cca aca act ggt tta tca gat gat agc att tta gac tta        240
   Gln Ser Ile Pro Thr Thr Gly Leu Ser Asp Asp Ser Ile Leu Asp Leu
   65                  70                  75                  80
   ttg caa aaa cta caa aat tta aaa cat tca gat tgg caa ggt ggt aaa        288
   Leu Gln Lys Leu Gln Asn Leu Lys His Ser Asp Trp Gln Gly Gly Lys
                   85                  90                  95
   gtc tca ggt gct gtt tac cat ggt ggt gat gat att att aag atc caa        336
   Val Ser Gly Ala Val Tyr His Gly Gly Asp Asp Ile Ile Lys Ile Gln
               100                 105                 110
   tct gat gct ttc aaa gtc ttt tgt gtt gct aat caa tta cat cca gac        384
   Ser Asp Ala Phe Lys Val Phe Cys Val Ala Asn Gln Leu His Pro Asp
           115                 120                 125
   gtt ttc cca ggt gtt cgt aaa atg gaa gct gaa gtt gtt gca atg act        432
   Val Phe Pro Gly Val Arg Lys Met Glu Ala Glu Val Val Ala Met Thr
       130                 135                 140
   ttg aaa tta ttc aat gca cca gaa tca ggt gtt ggt ggt acc agc tca        480
   Leu Lys Leu Phe Asn Ala Pro Glu Ser Gly Val Gly Gly Thr Ser Ser
   145                 150                 155                 160
   ggt ggt act gaa tcc tta tta ttg gct tgt ctt tct gct aaa gaa tat        528
   Gly Gly Thr Glu Ser Leu Leu Leu Ala Cys Leu Ser Ala Lys Glu Tyr
                   165                 170                 175
   ggt aaa cgt cat aaa ggt att gtt gaa cca gaa att att att cca gaa        576
   Gly Lys Arg His Lys Gly Ile Val Glu Pro Glu Ile Ile Ile Pro Glu
               180                 185                 190
   act gca cat gct ggt ttt gat aaa gct ggt tat tat ttt ggt atg aaa        624
   Thr Ala His Ala Gly Phe Asp Lys Ala Gly Tyr Tyr Phe Gly Met Lys
           195                 200                 205
   gtc cat cat gtt cca tta gat cca aag acc tat aaa gtt gat tta ggg        672
   Val His His Val Pro Leu Asp Pro Lys Thr Tyr Lys Val Asp Leu Gly
       210                 215                 220
   aaa tta aag aga tta atc aat aaa aac act gtt tta tta gct ggt tct        720
   Lys Leu Lys Arg Leu Ile Asn Lys Asn Thr Val Leu Leu Ala Gly Ser
   225                 230                 235                 240
   gca cca aat ttc cca cat ggt atc att gat gat att gaa tct att ggt        768
   Ala Pro Asn Phe Pro His Gly Ile Ile Asp Asp Ile Glu Ser Ile Gly
                   245                 250                 255
   gct cta ggt caa aaa tat aat atc cca gtt cat gtt gat tgt tgt tta        816
   Ala Leu Gly Gln Lys Tyr Asn Ile Pro Val His Val Asp Cys Cys Leu
               260                 265                 270
   ggt tca ttt att gtc tct tat atg gaa aaa gca ggt tat gaa tta cca        864
   Gly Ser Phe Ile Val Ser Tyr Met Glu Lys Ala Gly Tyr Glu Leu Pro
           275                 280                 285
   cct ttt gac ttt aga gtt cct ggt gtc act tca att tct tgt gat acc        912
   Pro Phe Asp Phe Arg Val Pro Gly Val Thr Ser Ile Ser Cys Asp Thr
       290                 295                 300
   cac aaa tac ggg ttt gca cca aaa ggt tct tca ata atc atg tat cgt          960
   His Lys Tyr Gly Phe Ala Pro Lys Gly Ser Ser Ile Ile Met Tyr Arg
   305                 310                 315                 320
   aat aat gct ctt aga gaa gca caa tat tat gtt aat gtt gac tgg gtt         1008
   Asn Asn Ala Leu Arg Glu Ala Gln Tyr Tyr Val Asn Val Asp Trp Val
                   325                 330                 335
   ggt ggt atc tat ggc tca cca act tta gct ggt agt aga cca ggt gct         1056
   Gly Gly Ile Tyr Gly Ser Pro Thr Leu Ala Gly Ser Arg Pro Gly Ala
               340                 345                 350
   atc att gtt ggt tgt tgg gca acc ttg atc aag att ggt gat gaa ggt         1104
   Ile Ile Val Gly Cys Trp Ala Thr Leu Ile Lys Ile Gly Asp Glu Gly
           355                 360                 365
   tac aag aaa tca tgt aaa gat att gtt gga gct gca aga aaa ttg aaa         1152
   Tyr Lys Lys Ser Cys Lys Asp Ile Val Gly Ala Ala Arg Lys Leu Lys
       370                 375                 380
   tta aga att caa aaa gaa ata cca gaa tta gaa atc att ggt gat cca         1200
   Leu Arg Ile Gln Lys Glu Ile Pro Glu Leu Glu Ile Ile Gly Asp Pro
   385                 390                 395                 400
   tta act tca gtt att tca ttc aaa tct gaa aaa att aat att tat gaa         1248
   Leu Thr Ser Val Ile Ser Phe Lys Ser Glu Lys Ile Asn Ile Tyr Glu
                   405                 410                 415
   tta tca gat ctc ttg agt tct aag gga tgg cac tta agt gca ttg caa         1296
   Leu Ser Asp Leu Leu Ser Ser Lys Gly Trp His Leu Ser Ala Leu Gln
               420                 425                 430
   aag cca gca gct tta cat ctt gca gtc act aga tta tca gtt cca gtt         1344
   Lys Pro Ala Ala Leu His Leu Ala Val Thr Arg Leu Ser Val Pro Val
           435                 440                 445
   att gat gaa tta gtt gat gaa ttg aaa aca gct gtt cac aaa ttg aga         1392
   Ile Asp Glu Leu Val Asp Glu Leu Lys Thr Ala Val His Lys Leu Arg
       450                 455                 460
   gat tca tct gct gct aaa ggt gat act gct gca tta tac ggt gtc gct         1440
   Asp Ser Ser Ala Ala Lys Gly Asp Thr Ala Ala Leu Tyr Gly Val Ala
   465                 470                 475                 480
   ggt agt gtt tcc acc act ggt gtt gtt gat cgt tta gtt gtt gga ttc         1488
   Gly Ser Val Ser Thr Thr Gly Val Val Asp Arg Leu Val Val Gly Phe
                   485                 490                 495
   tta gat aca cta tac aaa acc aaa taa                                     1515
   Leu Asp Thr Leu Tyr Lys Thr Lys
               500
<210> 10
   <211> 504
   <212> PRT
   <213> Pichia ciferrii
<400> 10
   Met Ala Val Asn Ile Thr Gly Tyr Gly Leu Ile Gly Tyr Leu Lys Ile
   1 5 10 15
   Val Tyr Asn Glu Leu Ala Lys Ala Val Phe Arg Thr Phe Leu Ser Leu
   20 25 30
   Pro Phe Val Lys Ser Lys Val Asp Ser Glu Val Arg Glu Asn Leu Asp
   35 40 45
   Lys Leu Glu Asp Ser Leu Ile Val Lys Thr Pro Asn Val Gln Asp Phe
   50 55 60
   Gln Ser Ile Pro Thr Thr Gly Leu Ser Asp Asp Ser Ile Leu Asp Leu
   65 70 75 80
   Leu Gln Lys Leu Gln Asn Leu Lys His Ser Asp Trp Gln Gly Gly Lys
   85 90 95
   Val Ser Gly Ala Val Tyr His Gly Gly Asp Asp Ile Ile Lys Ile Gln
   100 105 110
   Ser Asp Ala Phe Lys Val Phe Cys Val Ala Asn Gln Leu His Pro Asp
   115 120 125
   Val Phe Pro Gly Val Arg Lys Met Glu Ala Glu Val Val Ala Met Thr
   130 135 140
   Leu Lys Leu Phe Asn Ala Pro Glu Ser Gly Val Gly Gly Thr Ser Ser
   145 150 155 160
   Gly Gly Thr Glu Ser Leu Leu Leu Ala Cys Leu Ser Ala Lys Glu Tyr
   165 170 175
   Gly Lys Arg His Lys Gly Ile Val Glu Pro Glu Ile Ile Ile Pro Glu
   180 185 190
   Thr Ala His Ala Gly Phe Asp Lys Ala Gly Tyr Tyr Phe Gly Met Lys
   195 200 205
   Val His His Val Pro Leu Asp Pro Lys Thr Tyr Lys Val Asp Leu Gly
   210 215 220
   Lys Leu Lys Arg Leu Ile Asn Lys Asn Thr Val Leu Leu Ala Gly Ser
   225 230 235 240
   Ala Pro Asn Phe Pro His Gly Ile Ile Asp Asp Ile Glu Ser Ile Gly
   245 250 255
   Ala Leu Gly Gln Lys Tyr Asn Ile Pro Val His Val Asp Cys Cys Leu
               260                 265                 270
   Gly Ser Phe Ile Val Ser Tyr Met Glu Lys Ala Gly Tyr Glu Leu Pro
           275                 280                 285
   Pro Phe Asp Phe Arg Val Pro Gly Val Thr Ser Ile Ser Cys Asp Thr
       290                 295                 300
   His Lys Tyr Gly Phe Ala Pro Lys Gly Ser Ser Ile Ile Met Tyr Arg
   305                 310                 315                 320
   Asn Asn Ala Leu Arg Glu Ala Gln Tyr Tyr Val Asn Val Asp Trp Val
                   325                 330                 335
   Gly Gly Ile Tyr Gly Ser Pro Thr Leu Ala Gly Ser Arg Pro Gly Ala
               340                 345                 350
   Ile Ile Val Gly Cys Trp Ala Thr Leu Ile Lys Ile Gly Asp Glu Gly
           355                 360                 365
   Tyr Lys Lys Ser Cys Lys Asp Ile Val Gly Ala Ala Arg Lys Leu Lys
       370                 375                 380
   Leu Arg Ile Gln Lys Glu Ile Pro Glu Leu Glu Ile Ile Gly Asp Pro
   385                 390                 395                 400
   Leu Thr Ser Val Ile Ser Phe Lys Ser Glu Lys Ile Asn Ile Tyr Glu
                   405                 410                 415
   Leu Ser Asp Leu Leu Ser Ser Lys Gly Trp His Leu Ser Ala Leu Gln
               420                 425                 430
   Lys Pro Ala Ala Leu His Leu Ala Val Thr Arg Leu Ser Val Pro Val
           435                 440                 445
   Ile Asp Glu Leu Val Asp Glu Leu Lys Thr Ala Val His Lys Leu Arg
       450                 455                 460
   Asp Ser Ser Ala Ala Lys Gly Asp Thr Ala Ala Leu Tyr Gly Val Ala
   465                 470                 475                 480
   Gly Ser Val Ser Thr Thr Gly Val Val Asp Arg Leu Val Val Gly Phe
                   485                 490                 495
   Leu Asp Thr Leu Tyr Lys Thr Lys
               500
<210> 11
   <211> 576
   <212> DNA
   <213> Pichia ciferrii
<220>
   <221> CDS
   <222> (1)..(576)
<400> 11
   atg act aca aca cat gaa cca att tct gtt gat gga tca tta tca cca        48
   Met Thr Thr Thr His Glu Pro Ile Ser Val Asp Gly Ser Leu Ser Pro
   1               5                   10                  15
   aat tca aat aca aat aat aat aat caa cat cgt cgt cgt tca tca tca        96
   Asn Ser Asn Thr Asn Asn Asn Asn Gln His Arg Arg Arg Ser Ser Ser
               20                  25                  30
   ata att tct cat gtt gaa cct gaa act ttt gaa gaa aaa att gat caa       144
   Ile Ile Ser His Val Glu Pro Glu Thr Phe Glu Glu Lys Ile Asp Gln
           35                  40                  45
   gat tca aca cca aat tta aat gca aat tgg gtt cat tca aaa ggt gct       192
   Asp Ser Thr Pro Asn Leu Asn Ala Asn Trp Val His Ser Lys Gly Ala
       50                  55                  60
   tgg tta gtt cat att gtt att ata tta tta tta aaa att ttc ttt gat       240
   Trp Leu Val His Ile Val Ile Ile Leu Leu Leu Lys Ile Phe Phe Asp
   65                  70                  75                  80
   tta ata cct ggt tta tca aat gaa att agt tgg tca tta aca aat gct       288
   Leu Ile Pro Gly Leu Ser Asn Glu Ile Ser Trp Ser Leu Thr Asn Ala
                   85                  90                  95
   aca tat gtt att ggt tca tat att atg ttt cat tta gtt aaa ggt acg       336
   Thr Tyr Val Ile Gly Ser Tyr Ile Met Phe His Leu Val Lys Gly Thr
               100                 105                 110
   cca ttt gaa ttt aat tca ggt gct tat gat aat tta aca atg tgg gaa       384
   Pro Phe Glu Phe Asn Ser Gly Ala Tyr Asp Asn Leu Thr Met Trp Glu
           115                 120                 125
   caa tta gat gag gga gat ttt tat aca cca agt aaa aaa ttc tta gtt       432
   Gln Leu Asp Glu Gly Asp Phe Tyr Thr Pro Ser Lys Lys Phe Leu Val
       130                 135                 140
   ggt gta cca att tgg tta ttt ctt tgt tca act cat tat agt cat tat       480
   Gly Val Pro Ile Trp Leu Phe Leu Cys Ser Thr His Tyr Ser His Tyr
   145                 150                 155                 160
   gat tta aaa tta ttt att ata aat tta tta att tgt gct gtt ggt gtt       528
   Asp Leu Lys Leu Phe Ile Ile Asn Leu Leu Ile Cys Ala Val Gly Val
                   165                 170                 175
   gta cca aaa att cca att ttt gat cgt tta aga att aca ttt ttt taa       576
   Val Pro Lys Ile Pro Ile Phe Asp Arg Leu Arg Ile Thr Phe Phe
               180                 185                 190
<210> 12
   <211> 191
   <212> PRT
   <213> Pichia ciferrii
<400> 12
   Met Thr Thr Thr His Glu Pro Ile Ser Val Asp Gly Ser Leu Ser Pro
   1               5                   10                  15
   Asn Ser Asn Thr Asn Asn Asn Asn Gln His Arg Arg Arg Ser Ser Ser
               20                  25                  30
   Ile Ile Ser His Val Glu Pro Glu Thr Phe Glu Glu Lys Ile Asp Gln
           35                  40                  45
   Asp Ser Thr Pro Asn Leu Asn Ala Asn Trp Val His Ser Lys Gly Ala
       50                  55                  60
   Trp Leu Val His Ile Val Ile Ile Leu Leu Leu Lys Ile Phe Phe Asp
   65                  70                  75                  80
   Leu Ile Pro Gly Leu Ser Asn Glu Ile Ser Trp Ser Leu Thr Asn Ala
                   85                  90                  95
   Thr Tyr Val Ile Gly Ser Tyr Ile Met Phe His Leu Val Lys Gly Thr
               100                 105                 110
   Pro Phe Glu Phe Asn Ser Gly Ala Tyr Asp Asn Leu Thr Met Trp Glu
           115                 120                 125
   Gln Leu Asp Glu Gly Asp Phe Tyr Thr Pro Ser Lys Lys Phe Leu Val
       130                 135                 140
   Gly Val Pro Ile Trp Leu Phe Leu Cys Ser Thr His Tyr Ser His Tyr
   145                 150                 155                 160
   Asp Leu Lys Leu Phe Ile Ile Asn Leu Leu Ile Cys Ala Val Gly Val
                   165                 170                 175
   Val Pro Lys Ile Pro Ile Phe Asp Arg Leu Arg Ile Thr Phe Phe
               180                 185                 190
<210> 13
   <211> 1713
   <212> DNA
   <213> Pichia ciferrii
<220>
   <221> CDS
   <222> (1)..(1713)
<400> 13
   atg aac gtc act gct aca act ata aca act tca aca aca aca att gca          48
   Met Asn Val Thr Ala Thr Thr Ile Thr Thr Ser Thr Thr Thr Ile Ala
   1               5                   10                  15
   tta caa gat att tgg aat aca act tct gat gtt gtt tct cgt tat tta          96
   Leu Gln Asp Ile Trp Asn Thr Thr Ser Asp Val Val Ser Arg Tyr Leu
               20                  25                  30
   ttc att ata tta aat tat att gaa tta ata cct ggt ggt tca att tta         144
   Phe Ile Ile Leu Asn Tyr Ile Glu Leu Ile Pro Gly Gly Ser Ile Leu
           35                  40                  45
   gtt cgt tat ata aaa tct tct cat aaa aat gat cca att aga act tta         192
   Val Arg Tyr Ile Lys Ser Ser His Lys Asn Asp Pro Ile Arg Thr Leu
       50                  55                  60
   ttt gaa att gct tta ttt att ttt gca att aga tat ttt act aca gca         240
   Phe Glu Ile Ala Leu Phe Ile Phe Ala Ile Arg Tyr Phe Thr Thr Ala
   65                  70                  75                  80
   aaa tat gaa aga tct aaa aaa gat cat att aaa ttg aaa aat tct gaa         288
   Lys Tyr Glu Arg Ser Lys Lys Asp His Ile Lys Leu Lys Asn Ser Glu
                   85                  90                  95
   att gat gaa tta att gat gat tgg atg ccg gaa cct tta gtt ttg gat         336
   Ile Asp Glu Leu Ile Asp Asp Trp Met Pro Glu Pro Leu Val Leu Asp
               100                 105                 110
   att agt cca aag gaa caa tgg caa tta aat tca att cca att gtt aaa         384
   Ile Ser Pro Lys Glu Gln Trp Gln Leu Asn Ser Ile Pro Ile Val Lys
           115                 120                 125
   ggt cca ata gat act aaa gtg aac cta gtt ggt gaa gaa ggt gac ttt         432
   Gly Pro Ile Asp Thr Lys Val Asn Leu Val Gly Glu Glu Gly Asp Phe
       130                 135                 140
   tta aat ttt gct tct tca aat ttt tta aat ttt ggt att aat cca att         480
   Leu Asn Phe Ala Ser Ser Asn Phe Leu Asn Phe Gly Ile Asn Pro Ile
   145                 150                 155                 160
   gtt aaa aat gaa tgt aaa aaa att att cat agt aat ggt gtt ggt gct         528
   Val Lys Asn Glu Cys Lys Lys Ile Ile His Ser Asn Gly Val Gly Ala
                   165                 170                 175
   tgt ggt cca cca aat ttt tat ggt aat caa gat att cat att aaa tta         576
   Cys Gly Pro Pro Asn Phe Tyr Gly Asn Gln Asp Ile His Ile Lys Leu
               180                 185                 190
   gaa aat gat tta gca aaa ttt ttc gaa gtt ggt ggt gct gta tta tat         624
   Glu Asn Asp Leu Ala Lys Phe Phe Glu Val Gly Gly Ala Val Leu Tyr
           195                 200                 205
   ggt caa gat ttt tgt act gca ggt tca gtt tta cca agt ttt tta aaa         672
   Gly Gln Asp Phe Cys Thr Ala Gly Ser Val Leu Pro Ser Phe Leu Lys
       210                 215                 220
   aga ggt gat ttt gtt att gct gat gct tca tca aat gtt gca att caa         720
   Arg Gly Asp Phe Val Ile Ala Asp Ala Ser Ser Asn Val Ala Ile Gln
   225                 230                 235                 240
   aaa gct tta caa tta tca aga tgt gaa att tat tgg ttt aat cat aat         768
   Lys Ala Leu Gln Leu Ser Arg Cys Glu Ile Tyr Trp Phe Asn His Asn
                   245                 250                 255
   gat ttg gat cat tta gaa gaa att tta att gat tta caa aaa aat att          816
   Asp Leu Asp His Leu Glu Glu Ile Leu Ile Asp Leu Gln Lys Asn Ile
               260                 265                 270
   ttt aaa ttt gaa aaa cca att tca aga aaa ttt att gtt act gaa ggt          864
   Phe Lys Phe Glu Lys Pro Ile Ser Arg Lys Phe Ile Val Thr Glu Gly
           275                 280                 285
   att ttt gca aat aaa ggt gat tca cca tat tta cca aga tta att gaa          912
   Ile Phe Ala Asn Lys Gly Asp Ser Pro Tyr Leu Pro Arg Leu Ile Glu
       290                 295                 300
   tta aag aaa aaa ttt aaa ttt aga tta ttt ttg gat gaa tct tta tct          960
   Leu Lys Lys Lys Phe Lys Phe Arg Leu Phe Leu Asp Glu Ser Leu Ser
   305                 310                 315                 320
   tta ggt gtt tta ggt aaa tct ggt aaa ggt tta gct gaa cat tat aat         1008
   Leu Gly Val Leu Gly Lys Ser Gly Lys Gly Leu Ala Glu His Tyr Asn
                   325                 330                 335
   att aaa aga tca gaa att gat gta act ata agt tca atg gct aat tca         1056
   Ile Lys Arg Ser Glu Ile Asp Val Thr Ile Ser Ser Met Ala Asn Ser
               340                 345                 350
   ttc tct tct tca ggt gct ttt tgt att ggt gat aaa gtt atg act tat         1104
   Phe Ser Ser Ser Gly Ala Phe Cys Ile Gly Asp Lys Val Met Thr Tyr
           355                 360                 365
   cat caa aga att ggt tca atg gct tat tgt ttt agt gct tca tta cct         1152
   His Gln Arg Ile Gly Ser Met Ala Tyr Cys Phe Ser Ala Ser Leu Pro
       370                 375                 380
   gct tat gtt gca aga gct aca tca gtt gca tta aga tta tta act gat         1200
   Ala Tyr Val Ala Arg Ala Thr Ser Val Ala Leu Arg Leu Leu Thr Asp
   385                 390                 395                 400
   tct caa gat tcc cag ggt gaa tca tca att gta aaa aaa tta caa tca         1248
   Ser Gln Asp Ser Gln Gly Glu Ser Ser Ile Val Lys Lys Leu Gln Ser
                   405                 410                 415
   aat aat tat caa tta ttt aat tta ttt aat aaa gat aga aaa tta agt         1296
   Asn Asn Tyr Gln Leu Phe Asn Leu Phe Asn Lys Asp Arg Lys Leu Ser
               420                 425                 430
   aaa tat tta aaa att ata tca aat gaa att tca cca att tta cat ttt         1344
   Lys Tyr Leu Lys Ile Ile Ser Asn Glu Ile Ser Pro Ile Leu His Phe
           435                 440                 445
   gaa att aat tca gat tta aga aaa ctt tta aat ttc cca att agt tat         1392
   Glu Ile Asn Ser Asp Leu Arg Lys Leu Leu Asn Phe Pro Ile Ser Tyr
       450                 455                 460
   aca ggt aaa gga tca gaa att gaa tat aaa aat aaa aaa gga att tct         1440
   Thr Gly Lys Gly Ser Glu Ile Glu Tyr Lys Asn Lys Lys Gly Ile Ser
   465                 470                 475                 480
   gat aaa ttt gtt gaa tca ttt aat tat gaa aat tta att ttt caa aaa         1488
   Asp Lys Phe Val Glu Ser Phe Asn Tyr Glu Asn Leu Ile Phe Gln Lys
                   485                 490                 495
   att ata aat tta tcc aag aaa caa ggt att tta ata aca aga tca att         1536
   Ile Ile Asn Leu Ser Lys Lys Gln Gly Ile Leu Ile Thr Arg Ser Ile
               500                 505                 510
   ttt aca att gaa caa gaa gct ctg cct ctg att cca aat tta aaa att      1584
   Phe Thr Ile Glu Gln Glu Ala Leu Pro Leu Ile Pro Asn Leu Lys Ile
           515                 520                 525
   cat tca aat gtt gat ttt act aag gat gaa att gaa aaa gtt tat aaa      1632
   His Ser Asn Val Asp Phe Thr Lys Asp Glu Ile Glu Lys Val Tyr Lys
       530                 535                 540
   att gtt tcc aaa gta att tta gat gtt ttt gaa aat tta act gtt gaa      1680
   Ile Val Ser Lys Val Ile Leu Asp Val Phe Glu Asn Leu Thr Val Glu
   545                 550                 555                 560
   tca tta tca tta tta act gaa gaa gtt att taa                          1713
   Ser Leu Ser Leu Leu Thr Glu Glu Val Ile
                   565                 570
<210> 14
   <211> 570
   <212> PRT
   <213> Pichia ciferrii
<400> 14
   Met Asn Val Thr Ala Thr Thr Ile Thr Thr Ser Thr Thr Thr Ile Ala
   1               5                   10                  15
   Leu Gln Asp Ile Trp Asn Thr Thr Ser Asp Val Val Ser Arg Tyr Leu
               20                  25                  30
   Phe Ile Ile Leu Asn Tyr Ile Glu Leu Ile Pro Gly Gly Ser Ile Leu
           35                  40                  45
   Val Arg Tyr Ile Lys Ser Ser His Lys Asn Asp Pro Ile Arg Thr Leu
       50                  55                  60
   Phe Glu Ile Ala Leu Phe Ile Phe Ala Ile Arg Tyr Phe Thr Thr Ala
   65                  70                  75                  80
   Lys Tyr Glu Arg Ser Lys Lys Asp His Ile Lys Leu Lys Asn Ser Glu
                   85                  90                  95
   Ile Asp Glu Leu Ile Asp Asp Trp Met Pro Glu Pro Leu Val Leu Asp
               100                 105                 110
   Ile Ser Pro Lys Glu Gln Trp Gln Leu Asn Ser Ile Pro Ile Val Lys
           115                 120                 125
   Gly Pro Ile Asp Thr Lys Val Asn Leu Val Gly Glu Glu Gly Asp Phe
       130                 135                 140
   Leu Asn Phe Ala Ser Ser Asn Phe Leu Asn Phe Gly Ile Asn Pro Ile
   145                 150                 155                 160
   Val Lys Asn Glu Cys Lys Lys Ile Ile His Ser Asn Gly Val Gly Ala
                   165                 170                 175
   Cys Gly Pro Pro Asn Phe Tyr Gly Asn Gln Asp Ile His Ile Lys Leu
               180                 185                 190
   Glu Asn Asp Leu Ala Lys Phe Phe Glu Val Gly Gly Ala Val Leu Tyr
           195                 200                 205
   Gly Gln Asp Phe Cys Thr Ala Gly Ser Val Leu Pro Ser Phe Leu Lys
       210                 215                 220
   Arg Gly Asp Phe Val Ile Ala Asp Ala Ser Ser Asn Val Ala Ile Gln
   225                 230                 235                 240
   Lys Ala Leu Gln Leu Ser Arg Cys Glu Ile Tyr Trp Phe Asn His Asn
                   245                 250                 255
   Asp Leu Asp His Leu Glu Glu Ile Leu Ile Asp Leu Gln Lys Asn Ile
               260                 265                 270
   Phe Lys Phe Glu Lys Pro Ile Ser Arg Lys Phe Ile Val Thr Glu Gly
           275                 280                 285
   Ile Phe Ala Asn Lys Gly Asp Ser Pro Tyr Leu Pro Arg Leu Ile Glu
       290                 295                 300
   Leu Lys Lys Lys Phe Lys Phe Arg Leu Phe Leu Asp Glu Ser Leu Ser
   305                 310                 315                 320
   Leu Gly Val Leu Gly Lys Ser Gly Lys Gly Leu Ala Glu His Tyr Asn
                   325                 330                 335
   Ile Lys Arg Ser Glu Ile Asp Val Thr Ile Ser Ser Met Ala Asn Ser
               340                 345                 350
   Phe Ser Ser Ser Gly Ala Phe Cys Ile Gly Asp Lys Val Met Thr Tyr
           355                 360                 365
   His Gln Arg Ile Gly Ser Met Ala Tyr Cys Phe Ser Ala Ser Leu Pro
       370                 375                 380
   Ala Tyr Val Ala Arg Ala Thr Ser Val Ala Leu Arg Leu Leu Thr Asp
   385                 390                 395                 400
   Ser Gln Asp Ser Gln Gly Glu Ser Ser Ile Val Lys Lys Leu Gln Ser
                   405                 410                 415
   Asn Asn Tyr Gln Leu Phe Asn Leu Phe Asn Lys Asp Arg Lys Leu Ser
               420                 425                 430
   Lys Tyr Leu Lys Ile Ile Ser Asn Glu Ile Ser Pro Ile Leu His Phe
           435                 440                 445
   Glu Ile Asn Ser Asp Leu Arg Lys Leu Leu Asn Phe Pro Ile Ser Tyr
       450                 455                 460
   Thr Gly Lys Gly Ser Glu Ile Glu Tyr Lys Asn Lys Lys Gly Ile Ser
   465                 470                 475                 480
   Asp Lys Phe Val Glu Ser Phe Asn Tyr Glu Asn Leu Ile Phe Gln Lys
                   485                 490                 495
   Ile Ile Asn Leu Ser Lys Lys Gln Gly Ile Leu Ile Thr Arg Ser Ile
               500                 505                 510
   Phe Thr Ile Glu Gln Glu Ala Leu Pro Leu Ile Pro Asn Leu Lys Ile
           515                 520                 525
   His Ser Asn Val Asp Phe Thr Lys Asp Glu Ile Glu Lys Val Tyr Lys
       530                 535                 540
   Ile Val Ser Lys Val Ile Leu Asp Val Phe Glu Asn Leu Thr Val Glu
   545                 550                 555                 560
   Ser Leu Ser Leu Leu Thr Glu Glu Val Ile
                   565                 570
<210> 15
   <211> 1689
   <212> DNA
   <213> Pichia ciferrii
<220>
   <221> CDS
   <222> (1)..(1689)
<400> 15
   atg tca ttg gta ata cct caa ata gat cta tca ggt ctt tcc atc gaa         48
   Met Ser Leu Val Ile Pro Gln Ile Asp Leu Ser Gly Leu Ser Ile Glu
   1               5                   10                  15
   gac aag aaa caa aat gaa ttc ggt gct cta act tca aat gaa tat cgt         96
   Asp Lys Lys Gln Asn Glu Phe Gly Ala Leu Thr Ser Asn Glu Tyr Arg
               20                  25                  30
   tac aaa aca att tca aga cag ggg aaa cca tta cct gat cca att gaa         144
   Tyr Lys Thr Ile Ser Arg Gln Gly Lys Pro Leu Pro Asp Pro Ile Glu
           35                  40                  45
   gat gaa cca cca tat cat gtc ctt ttc atc act tat tta aac tat tta         192
   Asp Glu Pro Pro Tyr His Val Leu Phe Ile Thr Tyr Leu Asn Tyr Leu
       50                  55                  60
   atc ttg att atc gtt ggt cat att aaa gat ttc aca ggt att ctg ttc         240
   Ile Leu Ile Ile Val Gly His Ile Lys Asp Phe Thr Gly Ile Leu Phe
   65                  70                  75                  80
   aac cca aaa aat tac caa gat tta tta gaa caa aat ggc ctt gct cca         288
   Asn Pro Lys Asn Tyr Gln Asp Leu Leu Glu Gln Asn Gly Leu Ala Pro
                   85                  90                  95
   tgg tat aat aaa ttt gaa agt ttt tat att cgt cgt atg aaa caa aaa         336
   Trp Tyr Asn Lys Phe Glu Ser Phe Tyr Ile Arg Arg Met Lys Gln Lys
               100                 105                 110
   att gat gat tgt ttt gca aga cca act tgt ggt gtc cca ggt aga tta         384
   Ile Asp Asp Cys Phe Ala Arg Pro Thr Cys Gly Val Pro Gly Arg Leu
           115                 120                 125
   atc act tgt att gat cgt gat gct cat gat tat aat tca tat ttt agt         432
   Ile Thr Cys Ile Asp Arg Asp Ala His Asp Tyr Asn Ser Tyr Phe Ser
       130                 135                 140
   tat cct ggt act act tca act tgt tta aat tta tca tca tat aat tat         480
   Tyr Pro Gly Thr Thr Ser Thr Cys Leu Asn Leu Ser Ser Tyr Asn Tyr
   145                 150                 155                 160
   ttg ggg ttt gca caa tct gaa ggg gca tgt act caa gcc gct tta gaa         528
   Leu Gly Phe Ala Gln Ser Glu Gly Ala Cys Thr Gln Ala Ala Leu Glu
                   165                 170                 175
   att ttg gat tat tat ggt gtt ggt tct ggt ggt cca aga aat gtt att         576
   Ile Leu Asp Tyr Tyr Gly Val Gly Ser Gly Gly Pro Arg Asn Val Ile
               180                 185                 190
   ggt act act gat tta cat tta aaa act gaa aaa act ata gca aaa ttt         624
   Gly Thr Thr Asp Leu His Leu Lys Thr Glu Lys Thr Ile Ala Lys Phe
           195                 200                 205
   att ggt aaa gat gat tca atc tta ttt tca atg ggg tat gca aca aat         672
   Ile Gly Lys Asp Asp Ser Ile Leu Phe Ser Met Gly Tyr Ala Thr Asn
       210                 215                 220
   gca agt tta ttt agt tct tta ttg gat aag aaa tca ctt gtt att tct         720
   Ala Ser Leu Phe Ser Ser Leu Leu Asp Lys Lys Ser Leu Val Ile Ser
   225                 230                 235                 240
   gat gaa tta aat cat gct tca att aga act ggt gtt aga tta tct ggt         768
   Asp Glu Leu Asn His Ala Ser Ile Arg Thr Gly Val Arg Leu Ser Gly
                   245                 250                 255
   tct aca gtt aaa act ttc cct cat aat aat atg att gcc ttg gaa aaa         816
   Ser Thr Val Lys Thr Phe Pro His Asn Asn Met Ile Ala Leu Glu Lys
               260                 265                 270
   att ctt aga gaa caa att tct caa ggt caa cca aga tct cat cgt cca         864
   Ile Leu Arg Glu Gln Ile Ser Gln Gly Gln Pro Arg Ser His Arg Pro
               275                 280                 285
   tgg aaa aaa atc att gtt gca gtt gaa ggg ctt tat tca atg gag ggt        912
   Trp Lys Lys Ile Ile Val Ala Val Glu Gly Leu Tyr Ser Met Glu Gly
           290                 295                 300
   aca atg gca aat tta cct gca tta att gaa tta aga aga aaa tat aaa        960
   Thr Met Ala Asn Leu Pro Ala Leu Ile Glu Leu Arg Arg Lys Tyr Lys
   305                 310                 315                 320
   ttt aat tta ttt gtt gat gaa gct cat tca att ggt gct att ggt cca       1008
   Phe Asn Leu Phe Val Asp Glu Ala His Ser Ile Gly Ala Ile Gly Pro
                   325                 330                 335
   tca ggt cgt ggt gtt tgt gat tat ttt ggt ata gat ccc tca aat gtt       1056
   Ser Gly Arg Gly Val Cys Asp Tyr Phe Gly Ile Asp Pro Ser Asn Val
               340                 345                 350
   gat tta tta atg ggg act tta act aaa tca ttt ggt gct gca ggt ggt       1104
   Asp Leu Leu Met Gly Thr Leu Thr Lys Ser Phe Gly Ala Ala Gly Gly
           355                 360                 365
   tat att gct ggt tca caa caa att ata aat cgt tta aaa tta aat att       1152
   Tyr Ile Ala Gly Ser Gln Gln Ile Ile Asn Arg Leu Lys Leu Asn Ile
       370                 375                 380
   aat tca caa aat tat gca gaa tct atc cct gca cct gtt ttg gca caa       1200
   Asn Ser Gln Asn Tyr Ala Glu Ser Ile Pro Ala Pro Val Leu Ala Gln
   385                 390                 395                 400
   att att tct tcg tta aat atc atc tcg ggt gat tta aat cct ggt gaa       1248
   Ile Ile Ser Ser Leu Asn Ile Ile Ser Gly Asp Leu Asn Pro Gly Glu
                   405                 410                 415
   ggt tcg gaa aga tta gaa aga att gct ttt aat tca cgt tat tta aga       1296
   Gly Ser Glu Arg Leu Glu Arg Ile Ala Phe Asn Ser Arg Tyr Leu Arg
               420                 425                 430
   tta ggt tta caa aga tta ggt ttt atc gta tac gga gtt gat gat tca       1344
   Leu Gly Leu Gln Arg Leu Gly Phe Ile Val Tyr Gly Val Asp Asp Ser
           435                 440                 445
   cca gtg att cca tta tta tta ttc gcc cca gcc aaa atg cca gca ttt       1392
   Pro Val Ile Pro Leu Leu Leu Phe Ala Pro Ala Lys Met Pro Ala Phe
       450                 455                 460
   tca cgt atg cta tat caa aga aaa att gca gtt gtt gtt gtt gga tac       1440
   Ser Arg Met Leu Tyr Gln Arg Lys Ile Ala Val Val Val Val Gly Tyr
   465                 470                 475                 480
   ccg gca act cca ctg act tca tca aga gtt cgt ctt tgt gtt tct gca       1488
   Pro Ala Thr Pro Leu Thr Ser Ser Arg Val Arg Leu Cys Val Ser Ala
                   485                 490                 495
   tct tta aca aaa gaa gat att gat tat ctt tta cgt cat tta tcc gag       1536
   Ser Leu Thr Lys Glu Asp Ile Asp Tyr Leu Leu Arg His Leu Ser Glu
               500                 505                 510
   gtg ggt gat aaa tta ttt tta aaa ttt agt tct ggt att gct ggt ggt       1584
   Val Gly Asp Lys Leu Phe Leu Lys Phe Ser Ser Gly Ile Ala Gly Gly
           515                 520                 525
   tct tta gat ggt tca cca cca aga tgg aat att gaa gat gtt ttg aaa       1632
   Ser Leu Asp Gly Ser Pro Pro Arg Trp Asn Ile Glu Asp Val Leu Lys
       530                 535                 540
   gag act cca aag gat tgt aaa gaa tct aaa tat ttt att gca act gca       1680
   Glu Thr Pro Lys Asp Cys Lys Glu Ser Lys Tyr Phe Ile Ala Thr Ala
   545                 550                 555                 560
   aat aat tga                                                           1689
   Asn Asn
<210> 16
   <211> 562
   <212> PRT
   <213> Pichia ciferrii
<400> 16
   Met Ser Leu Val Ile Pro Gln Ile Asp Leu Ser Gly Leu Ser Ile Glu
   1               5                   10                  15
   Asp Lys Lys Gln Asn Glu Phe Gly Ala Leu Thr Ser Asn Glu Tyr Arg
               20                  25                  30
   Tyr Lys Thr Ile Ser Arg Gln Gly Lys Pro Leu Pro Asp Pro Ile Glu
           35                  40                  45
   Asp Glu Pro Pro Tyr His Val Leu Phe Ile Thr Tyr Leu Asn Tyr Leu
       50                  55                  60
   Ile Leu Ile Ile Val Gly His Ile Lys Asp Phe Thr Gly Ile Leu Phe
   65                  70                  75                  80
   Asn Pro Lys Asn Tyr Gln Asp Leu Leu Glu Gln Asn Gly Leu Ala Pro
                   85                  90                  95
   Trp Tyr Asn Lys Phe Glu Ser Phe Tyr Ile Arg Arg Met Lys Gln Lys
               100                 105                 110
   Ile Asp Asp Cys Phe Ala Arg Pro Thr Cys Gly Val Pro Gly Arg Leu
           115                 120                 125
   Ile Thr Cys Ile Asp Arg Asp Ala His Asp Tyr Asn Ser Tyr Phe Ser
       130                 135                 140
   Tyr Pro Gly Thr Thr Ser Thr Cys Leu Asn Leu Ser Ser Tyr Asn Tyr
   145                 150                 155                 160
   Leu Gly Phe Ala Gln Ser Glu Gly Ala Cys Thr Gln Ala Ala Leu Glu
                   165                 170                 175
   Ile Leu Asp Tyr Tyr Gly Val Gly Ser Gly Gly Pro Arg Asn Val Ile
               180                 185                 190
   Gly Thr Thr Asp Leu His Leu Lys Thr Glu Lys Thr Ile Ala Lys Phe
           195                 200                 205
   Ile Gly Lys Asp Asp Ser Ile Leu Phe Ser Met Gly Tyr Ala Thr Asn
       210                 215                 220
   Ala Ser Leu Phe Ser Ser Leu Leu Asp Lys Lys Ser Leu Val Ile Ser
   225                 230                 235                 240
   Asp Glu Leu Asn His Ala Ser Ile Arg Thr Gly Val Arg Leu Ser Gly
                   245                 250                 255
   Ser Thr Val Lys Thr Phe Pro His Asn Asn Met Ile Ala Leu Glu Lys
               260                 265                 270
   Ile Leu Arg Glu Gln Ile Ser Gln Gly Gln Pro Arg Ser His Arg Pro
           275                 280                 285
   Trp Lys Lys Ile Ile Val Ala Val Glu Gly Leu Tyr Ser Met Glu Gly
       290                 295                 300
   Thr Met Ala Asn Leu Pro Ala Leu Ile Glu Leu Arg Arg Lys Tyr Lys
   305                 310                 315                 320
   Phe Asn Leu Phe Val Asp Glu Ala His Ser Ile Gly Ala Ile Gly Pro
                   325                 330                 335
   Ser Gly Arg Gly Val Cys Asp Tyr Phe Gly Ile Asp Pro Ser Asn Val
               340                 345                 350
   Asp Leu Leu Met Gly Thr Leu Thr Lys Ser Phe Gly Ala Ala Gly Gly
           355                 360                 365
   Tyr Ile Ala Gly Ser Gln Gln Ile Ile Asn Arg Leu Lys Leu Asn Ile
       370                 375                 380
   Asn Ser Gln Asn Tyr Ala Glu Ser Ile Pro Ala Pro Val Leu Ala Gln
   385                 390                 395                 400
   Ile Ile Ser Ser Leu Asn Ile Ile Ser Gly Asp Leu Asn Pro Gly Glu
                   405                 410                 415
   Gly Ser Glu Arg Leu Glu Arg Ile Ala Phe Asn Ser Arg Tyr Leu Arg
               420                 425                 430
   Leu Gly Leu Gln Arg Leu Gly Phe Ile Val Tyr Gly Val Asp Asp Ser
   435 440 445
   Pro Val Ile Pro Leu Leu Leu Phe Ala Pro Ala Lys Met Pro Ala Phe
   450 455 460
   Ser Arg Met Leu Tyr Gln Arg Lys Ile Ala Val Val Val Val Gly Tyr
   465 470 475 480
   Pro Ala Thr Pro Leu Thr Ser Ser Arg Val Arg Leu Cys Val Ser Ala
   485 490 495
   Ser Leu Thr Lys Glu Asp Ile Asp Tyr Leu Leu Arg His Leu Ser Glu
   500 505 510
   Val Gly Asp Lys Leu Phe Leu Lys Phe Ser Ser Gly Ile Ala Gly Gly
   515 520 525
   Ser Leu Asp Gly Ser Pro Pro Arg Trp Asn Ile Glu Asp Val Leu Lys
   530 535 540
   Glu Thr Pro Lys Asp Cys Lys Glu Ser Lys Tyr Phe Ile Ala Thr Ala
   545 550 555 560
   Asn Asn
<210> 17
   <211> 978
   <212> DNA
   <213> Pichia ciferrii
<220>
   <221> CDS
   <222> (1)..(978)
<400> 17
   atg agc tct cat cag ttt ttg atc aac caa aca act ttg gcg gct cca 48
   Met Ser Ser His Gln Phe Leu Ile Asn Gln Thr Thr Leu Ala Ala Pro
   1 5 10 15
   cct gtt cat ttg gtg gag aaa cca agt ttg att aat ggc ata ccg gat 96
   Pro Val His Leu Val Glu Lys Pro Ser Leu Ile Asn Gly Ile Pro Asp
   20 25 30
   aac att tta gcc ttg att gca cct gtt ata gct tat tat tca tat tca 144
   Asn Ile Leu Ala Leu Ile Ala Pro Val Ile Ala Tyr Tyr Ser Tyr Ser
   35 40 45
   gga ttt ttc tat gtg att gat act tta gaa att gca gaa ctt tat aga 192
   Gly Phe Phe Tyr Val Ile Asp Thr Leu Glu Ile Ala Glu Leu Tyr Arg
   50 55 60
   att cat cca cct gaa gaa gtt agt tca aga aat aaa gct aca aaa ttt      240
   Ile His Pro Pro Glu Glu Val Ser Ser Arg Asn Lys Ala Thr Lys Phe
   65                  70                  75                  80
   gat gtt tta aaa gat gtt gtt tta caa cat ttt ata cag agt gtt gtt      288
   Asp Val Leu Lys Asp Val Val Leu Gln His Phe Ile Gln Ser Val Val
                   85                  90                  95
   ggt tat atc ttt aca tat ttt gat cca att caa tat act ggt gat gaa      336
   Gly Tyr Ile Phe Thr Tyr Phe Asp Pro Ile Gln Tyr Thr Gly Asp Glu
               100                 105                 110
   gaa tat caa gct tgg aaa tta caa caa act tta cca ttt tta cca ttt      384
   Glu Tyr Gln Ala Trp Lys Leu Gln Gln Thr Leu Pro Phe Leu Pro Phe
           115                 120                 125
   gat gtt gca tat tat tgg aat atg tat ggt tgg agt tgt ttg aaa att      432
   Asp Val Ala Tyr Tyr Trp Asn Met Tyr Gly Trp Ser Cys Leu Lys Ile
       130                 135                 140
   ggt ctt gca ttt tta att att gat tca tgg caa tat tgg tta cat aga      480
   Gly Leu Ala Phe Leu Ile Ile Asp Ser Trp Gln Tyr Trp Leu His Arg
   145                 150                 155                 160
   att atg cat tta aac aag aca tta tac aaa aga ttc cat tca aga cat      528
   Ile Met His Leu Asn Lys Thr Leu Tyr Lys Arg Phe His Ser Arg His
                   165                 170                 175
   cat cgt ctt tat gtc cca tat gct ttt ggt gct tta tat aat gat cca      576
   His Arg Leu Tyr Val Pro Tyr Ala Phe Gly Ala Leu Tyr Asn Asp Pro
               180                 185                 190
   ttt gaa ggg ttt tta ttg gat acc tta ggt acc ggt att gct gca att      624
   Phe Glu Gly Phe Leu Leu Asp Thr Leu Gly Thr Gly Ile Ala Ala Ile
           195                 200                 205
   gtt act caa tta act cca aga gaa tct att gtt tta tat aca ttt tca      672
   Val Thr Gln Leu Thr Pro Arg Glu Ser Ile Val Leu Tyr Thr Phe Ser
       210                 215                 220
   act ttg aaa act gtt gat gat cat tgt ggt tat tca tta cct tat gat      720
   Thr Leu Lys Thr Val Asp Asp His Cys Gly Tyr Ser Leu Pro Tyr Asp
   225                 230                 235                 240
   cct ttc caa att ttg ttc cca aat aac tca att tat cat gat att cat      768
   Pro Phe Gln Ile Leu Phe Pro Asn Asn Ser Ile Tyr His Asp Ile His
                   245                 250                 255
   cat caa caa ttt ggt atc aag acc aat ttt tca caa cct ttc ttt aca      816
   His Gln Gln Phe Gly Ile Lys Thr Asn Phe Ser Gln Pro Phe Phe Thr
               260                 265                 270
   cat tgg gat gtt ttc agt aat aca aga tat aaa gaa att gat gaa tac      864
   His Trp Asp Val Phe Ser Asn Thr Arg Tyr Lys Glu Ile Asp Glu Tyr
           275                 280                 285
   aga gaa aag caa aaa gct att aca att gcc aaa tat aaa gag ttt tta      912
   Arg Glu Lys Gln Lys Ala Ile Thr Ile Ala Lys Tyr Lys Glu Phe Leu
       290                 295                 300
   cat gat cgt gaa att gca aaa caa aag aag aag gct gaa att tat aaa      960
   His Asp Arg Glu Ile Ala Lys Gln Lys Lys Lys Ala Glu Ile Tyr Lys
   305                 310                 315                 320
   gat aag aaa act gat tga                                                   978
   Asp Lys Lys Thr Asp
                   325
<210> 18
   <211> 325
   <212> PRT
   <213> Pichia ciferrii
<400> 18
   Met Ser Ser His Gln Phe Leu Ile Asn Gln Thr Thr Leu Ala Ala Pro
   1               5                   10                  15
   Pro Val His Leu Val Glu Lys Pro Ser Leu Ile Asn Gly Ile Pro Asp
               20                  25                  30
   Asn Ile Leu Ala Leu Ile Ala Pro Val Ile Ala Tyr Tyr Ser Tyr Ser
           35                  40                  45
   Gly Phe Phe Tyr Val Ile Asp Thr Leu Glu Ile Ala Glu Leu Tyr Arg
       50                  55                  60
   Ile His Pro Pro Glu Glu Val Ser Ser Arg Asn Lys Ala Thr Lys Phe
   65                  70                  75                  80
   Asp Val Leu Lys Asp Val Val Leu Gln His Phe Ile Gln Ser Val Val
                   85                  90                  95
   Gly Tyr Ile Phe Thr Tyr Phe Asp Pro Ile Gln Tyr Thr Gly Asp Glu
               100                 105                 110
   Glu Tyr Gln Ala Trp Lys Leu Gln Gln Thr Leu Pro Phe Leu Pro Phe
           115                 120                 125
   Asp Val Ala Tyr Tyr Trp Asn Met Tyr Gly Trp Ser Cys Leu Lys Ile
       130                 135                 140
   Gly Leu Ala Phe Leu Ile Ile Asp Ser Trp Gln Tyr Trp Leu His Arg
   145                 150                 155                 160
   Ile Met His Leu Asn Lys Thr Leu Tyr Lys Arg Phe His Ser Arg His
                   165                 170                 175
   His Arg Leu Tyr Val Pro Tyr Ala Phe Gly Ala Leu Tyr Asn Asp Pro
               180                 185                 190
   Phe Glu Gly Phe Leu Leu Asp Thr Leu Gly Thr Gly Ile Ala Ala Ile
           195                 200                 205
   Val Thr Gln Leu Thr Pro Arg Glu Ser Ile Val Leu Tyr Thr Phe Ser
       210                 215                 220
   Thr Leu Lys Thr Val Asp Asp His Cys Gly Tyr Ser Leu Pro Tyr Asp
   225                 230                 235                 240
   Pro Phe Gln Ile Leu Phe Pro Asn Asn Ser Ile Tyr His Asp Ile His
                   245                 250                 255
   His Gln Gln Phe Gly Ile Lys Thr Asn Phe Ser Gln Pro Phe Phe Thr
               260                 265                 270
   His Trp Asp Val Phe Ser Asn Thr Arg Tyr Lys Glu Ile Asp Glu Tyr
           275                 280                 285
   Arg Glu Lys Gln Lys Ala Ile Thr Ile Ala Lys Tyr Lys Glu Phe Leu
       290                 295                 300
   His Asp Arg Glu Ile Ala Lys Gln Lys Lys Lys Ala Glu Ile Tyr Lys
   305                 310                 315                 320
   Asp Lys Lys Thr Asp
                   325
<210> 19
   <211> 1681
   <212> DNA
   <213> Artificial
<220>
   <223> Kassette
<400> 19
   gaaattaata cgactcacta tagggagacc ggcagatccg cggccgcata ggccactagt      60
   ggatctgata tcatcgatga attcgagctc ataacttcgt atagcataca ttatacgaag     120
   ttattcgaca ctggatggcg gcgttagtat cgaatcgaca gcagtatagc gaccagcatt     180
   cacatacgat tgacgcatga tattactttc tgcgcactta acttcgcatc tgggcagatg     240
   atgtcgaggc gaaaaaaaat ataaatcacg ctaacatttg attaaaatag aacaactaca     300
   atataaaaaa actatacaaa tgacaagttc ttgaaaacaa gaatcttttt attgtcagta     360
   ctgattatta tggacatggc attgacatat ataaagcttg ttcaccatct gaagcagtac     420
   catcatataa agcagtatct aaaccacata atgtgaaacc cattcttcta taagcatgaa     480
   tagctggagc attaacatta gtaacttcta accataaatg accagcacca cgttctctag     540
   caaattcagt agctaaaccc attaaagctc taccaacacc atgacctcta tgttctggag     600
   caacttcaat atcttcaaca gttaatcttc tattccaacc tgaatatgaa acaacaacaa     660
   aaccagctaa gtctccgtca tcaccataag caacaaaagt tcttgaatct ggatcaccat     720
   cttcaccgtc atcagactcg tcatctgatt catcatctgg aaaaacttta gttaatggtg     780
   gatcaactgg aacttctctt aaagtaaaac catcaccagt agcagtaact ctaaaaacag     840
   tatcagtagt aaatgaacca tctaaagctt caatagcttc agcatcacct ggaactgaag     900
   ttctatatct ataagcagta tcatctaaag tagtacccat tgataataaa gttgattttg     960
   aagtttggaa agtagtttct ggaacttcta attcataacc ttcaaatgat gaagctggta    1020
   agttaatagt gacaatctgt gtgaaaacgg gttagtaatt aaacattgtc tagtgtttcc    1080
   cactgatttg gattgaaaat ttggtgattt gtggttgtat agatctaaat cttgattgtc    1140
   ccctatcttt cctagatatc aaaaaaacaa tcacaaaaca atcaaagaac caattaaaat    1200
   ccaatcaatt catctccatt atccacaatt catcatcgat ccaaaaatat aataacaatc    1260
   tacttacttc atcatcttgg ttggcttcag tggccatagt tctggcaact ctttgagttg    1320
   atctcaaaga agttgtgttt gaaagaggac gaacaatatt cttcaacatc atctttgtat    1380
   agtagtctga actcctccgg gaaagtttag ttgtgttgaa tatttagttg aaaatggggg    1440
   agaattgcaa acctctaata aaagttgaat acttctacta ttttcaaacc aaacaaatta    1500
   tcaattgaat gtattattga attttgaatt caaaatcgat aaatttactt ttcgtttttt    1560
   cgcatcaggt gtttgaaaat ggccggtgcg tcgcgaaccg ggcaaattta gagcacaata    1620
   acttcgtata gcatacatta tacgaagtta tctgcaggtt acccagtggt acgaagcgcc    1680
   a                                                                    1681
<210> 20
   <211> 3872
   <212> DNA
   <213> Artificial
<220>
   <223> Kassette
<400> 20
   tgagcaaaag gccagcaaaa ggccaggaac cgtaaaaagg ccgcgttgct ggcgtttttc      60
   cataggctcc gcccccctga cgagcatcac aaaaatcgac gctcaagtca gaggtggcga     120
   aacccgacag gactataaag ataccaggcg tttccccctg gaagctccct cgtgcgctct     180
   cctgttccga ccctgccgct taccggatac ctgtccgcct ttctcccttc gggaagcgtg     240
   gcgctttctc aatgctcacg ctgtaggtat ctcagttcgg tgtaggtcgt tcgctccaag     300
   ctgggctgtg tgcacgaacc ccccgttcag cccgaccgct gcgccttatc cggtaactat     360
   cgtcttgagt ccaacccggt aagacacgac ttatcgccac tggcagcagc cactggtaac     420
   aggattagca gagcgaggta tgtaggcggt gctacagagt tcttgaagtg gtggcctaac      480
   tacggctaca ctagaaggac agtatttggt atctgcgctc tgctgaagcc agttaccttc      540
   ggaaaaagag ttggtagctc ttgatccggc aaacaaacca ccgctggtag cggtggtttt      600
   tttgtttgca agcagcagat tacgcgcaga aaaaaaggat ctcaagaaga tcctttgatc      660
   ttttctacgg ggtctgacgc tcagtggaac gaaaactcac gttaagggat tttggtcatg      720
   agattatcaa aaaggatctt cacctagatc cttttaaatt aaaaatgaag ttttaaatca      780
   atctaaagta tatatgagta aacttggtct gacagttacc aatgcttaat cagtgaggca      840
   cctatctcag cgatctgtct atttcgttca tccatagttg cctgactccc cgtcgtgtag      900
   ataactacga tacgggaggg cttaccatct ggccccagtg ctgcaatgat accgcgagac      960
   ccacgctcac cggctccaga tttatcagca ataaaccagc cagccggaag ggccgagcgc     1020
   agaagtggtc ctgcaacttt atccgcctcc atccagtcta ttaattgttg ccgggaagct     1080
   agagtaagta gttcgccagt taatagtttg cgcaacgttg ttgccattgc tacaggcatc     1140
   gtggtgtcac gctcgtcgtt tggtatggct tcattcagct ccggttccca acgatcaagg     1200
   cgagttacat gatcccccat gttgtgcaaa aaagcggtta gctccttcgg tcctccgatc     1260
   gttgtcagaa gtaagttggc cgcagtgtta tcactcatgg ttatggcagc actgcataat     1320
   tctcttactg tcatgccatc cgtaagatgc ttttctgtga ctggtgagta ctcaaccaag     1380
   tcattctgag aatagtgtat gcggcgaccg agttgctctt gcccggcgtc aatacgggat     1440
   aataccgcgc cacatagcag aactttaaaa gtgctcatca ttggaaaacg ttcttcgggg     1500
   cgaaaactct caaggatctt accgctgttg agatccagtt cgatgtaacc cactcgtgca     1560
   cccaactgat cttcagcatc ttttactttc accagcgttt ctgggtgagc aaaaacagga     1620
   aggcaaaatg ccgcaaaaaa gggaataagg gcgacacgga aatgttgaat actcatactc     1680
   ttcctttttc aatattattg aagcatttat cagggttatt gtctcatgag cggatacata     1740
   tttgaatgta tttagaaaaa taaacaaata ggggttccgc gcacatttcc ccgaaaagtg     1800
   ccacctgacg tctaagaaac cattattatc atgacattaa cctataaaaa taggcgtatc     1860
   acgaggccct ttcgtctcgc gcgtttcggt gatgacggtg aaaacctctg acacatgctg     1920
   tgctctaaat ttgcccggtt cgcgacgcac cggccatttt caaacacctg atgcgaaaaa     1980
   acgaaaagta aatttatcga ttttgaattc aaaattcaat aatacattca attgataatt     2040
   tgtttggttt gaaaatagta gaagtattca acttttatta gaggtttgca attctccccc     2100
   attttcaact aaatattcaa cacaactaaa ctttcccgga ggagttcaga ctactataca     2160
   aagatgatgt tgaagaatat tgttcgtcct ctttcaaaca caacttcttt gagatcaact     2220
   caaagagttg ccagaactat ggccactgaa gccaaccaag atgatgaagt aagtagattg     2280
   ttattatatt tttggatcga tgatgaattg tggataatgg agatgaattg attggatttt     2340
   aattggttct ttgattgttt tgtgattgtt tttttgatat ctaggaaaga taggggacaa 2400
   tcaagattta gatctataca accacaaatc accaaatttt caatccaaat cagtgggaaa 2460
   cactagacaa tgtttaatta ctaacccgtt ttcacacaga ttgtcactat taacttacca 2520
   gcttcatcat ttgaaggtta tgaattagaa gttccagaaa ctactttcca aacttcaaaa 2580
   tcaactttat tatcaatgtc aaatttatta actgttcatc aaaatttacc agctttacca 2640
   gttgatgcta cttcagatga agttagaaaa aatttaatgg atatgtttag agatagacaa 2700
   gctttttcag aacatacttg gaaaatgtta ttatcagttt gtagatcatg ggctgcttgg 2760
   tgtaaattaa ataatagaaa atggtttcca gctgaaccag aagatgttag agattactta 2820
   ttatatttac aagctagagg tttagctgtt aaaactattc aacaacactt aggacaatta 2880
   aatatgttac atcgtagatc aggtttacca agaccatcag attcaaatgc tgtttcatta 2940
   gttatgagaa gaattagaaa agaaaatgtt gatgctggtg aaagagctaa acaagcttta 3000
   gcttttgaaa gaactgattt tgatcaagtt agatcattaa tggaaaattc agatagatgt 3060
   caagatatta gaaacttagc ttttttaggt attgcttata atactttatt aagaattgct 3120
   gaaattgcta gaattagagt taaagatatt tcaagaactg atggtggtag aatgttaatt 3180
   catattggta gaactaaaac tttagtttca actgctggtg ttgaaaaagc tttatcatta 3240
   ggtgttacta aattagttga aagatggatt tcagtttcag gtgttgctga tgatccaaat 3300
   aattacttat tctgtagagt tagaaaaaat ggtgttgctg ctccatcagc tacttcacaa 3360
   ttatcaacta gagctttaga aggtattttt gaagctactc atcgtttaat ctatggtgct 3420
   aaagatgatt caggtcaaag atacttagct tggagtggac attcagctag agttggtgct 3480
   gctagagata tggctagagc tggtgtttca attccagaaa ttatgcaagc tggaggatgg 3540
   actaatgtta atattgttat gaattatatt agaaacttag attcagaaac tggtgctatg 3600
   gttcgtttat tagaagatgg tgattaatca gtactgacaa taaaaagatt cttgttttca 3660
   agaacttgtc atttgtatag tttttttata ttgtagttgt tctattttaa tcaaatgtta 3720
   gcgtgattta tatttttttt cgcctcgaca tcatctgccc agatgcgaag ttaagtgcgc 3780
   agaaagtaat atcatgcgtc aatcgtatgt gaatgctggt cgctatactg ctgtcgattc 3840
   gatactaacg ccgccatcca gtgtcgagca tg 3872
<210> 21
   <211> 584
   <212> DNA
   <213> Pichia ciferrii
<400> 21
   cagatcaaac cacatcatga gcttcaattg ataaacatga gaacatgaga ttccaattct 60
   ttaacgttgt gcgtggcttg acggatccta tatacgctaa cacgctaaac gctaaacgtc 120
   aagacgagaa ccaacccgca tcttgccatt gcaaggccaa ttcaagagat gttttctgga 180
   taattagtgt aaagtgttca attgtgctcg aggaatccaa ccattataac ctcatccttt 240
   tgagaacaat agatttggta cttattgtta taaattctat cgcaacttgt cctgtctaac 300
   ggtgggaaat tggcatcacc tggtgatgtt ttggccaaca cctgagccat tacccgctgc 360
   ttctcagcac catattttgt taaaccactt gatctcacca tacaacgaca ccaccgggta 420
   ccacacttgc gttgggcaga aattctcaat tcgccaattc caattgtagt ataaatacat 480
   catttattcc cttttatcag aaactataag taataataga agaattcttt tttcctttct 540
   ctatcaattg tactcaattc gataaaacat atacacatta caca 584
<210> 22
   <211> 420
   <212> DNA
   <213> Pichia ciferrii
<400> 22
   cggaccgtta attaccaaca atctcaattg tacaacatag tgttaaaaca ggataacttg 60
   atgattatat gtgatattaa gttcaaacaa gtaccaataa atagataatt aatagctcta 120
   taatatatca tttaattgaa ttaatatcaa tagttgttgt ttaattatcc ctagttttct 180
   ggttaaagtt acaccatcag atggttcacc accaatgttg ttcaaaccat ttccactcaa 240
   ctgacgtttc aagaacatca cctgaaaaaa aaaaattcat cacacattgg gagaaattgg 300
   gagaattgta tataaggagt tgaaatcgct aatattttta tacttctact cacttgtttt 360
   aattctacat cagtatttta taatacaaaa acaaacaaac aaacaaataa ttaattaaca 420
<210> 23
   <211> 19
   <212> DNA
   <213> Artificial Sequence
<220>
   <223> Primer
<400> 23
   gcttccggct cctatgttg 19
<210> 24
   <211> 21
   <212> DNA
   <213> Artificial Sequence
<220>
   <223> Primer
<400> 24
   accctatgcg gtgtgaaata c 21
<210> 25
   <211> 25
   <212> DNA
   <213> Artificial Sequence
<220>
   <223> Primer
<400> 25
   gccaatactt cacaatgttc gaatc 25
<210> 26
   <211> 23
   <212> DNA
   <213> Artificial Sequence
<220>
   <223> Primer
<400> 26
   cgtgaatgta agcgtgacat aac 23
<210> 27
   <211> 57
   <212> DNA
   <213> Artificial Sequence
<220>
   <223> Primer
<400> 27
   caaaaagtta acatgcatca ccatcaccat cacactaacc caactaggct cattaac 57
<210> 28
   <211> 58
   <212> DNA
   <213> Artificial Sequence
<220>
   <223> Primer
<400> 28
   gttatctgca ggttacccag tggtacgaag cgccatcagc catttctgga tcaatttc 58
<210> 29
   <211> 58
   <212> DNA
   <213> Artificial Sequence
<220>
   <223> Primer
<400> 29
   tgccggtctc cctatagtga gtcgtattaa tttcatccag ttccaggtga attataag 58
<210> 30
   <211> 58
   <212> DNA
   <213> Artificial Sequence
<220>
   <223> Primer
<400> 30
   taactaatta catgactcga ggtcgacggt atcccatact atgcttggca tcttaaac 58
<210> 31
   <211> 22
   <212> DNA
   <213> Artificial Sequence
<220>
   <223> Primer
<400> 31
   ttgatagggc aaattctcca ac 22
<210> 32
   <211> 20
   <212> DNA
   <213> Artificial Sequence
<220>
   <223> Primer
<400> 32
   ttcacctgga taaccttctg 20
<210> 33
   <211> 54
   <212> DNA
   <213> Artificial Sequence
<220>
   <223> Primer
<400> 33
   caaaaagtta acatgcatca ccatcaccat cacatgtcct tgcaggtggt attc 54
<210> 34
   <211> 55
   <212> DNA
   <213> Artificial Sequence
<220>
   <223> Primer
<400> 34
   ttatctgcag gttacccagt ggtacgaagc gccaggtaaa gcgtatggca tgttg 55
<210> 35
   <211> 57
   <212> DNA
   <213> Artificial Sequence
<220>
   <223> Primer
<400> 35
   ctgccggtct ccctatagtg agtcgtatta atttcgctgg tgaattccca ttatctg 57
<210> 36
   <211> 58
   <212> DNA
   <213> Artificial Sequence
<220>
   <223> Primer
<400> 36
   taactaatta catgactcga ggtcgacggt atccataacc atctaaagca ttatagtc 58
<210> 37
   <211> 22
   <212> DNA
   <213> Artificial Sequence
<220>
   <223> Primer
<400> 37
   aagtttcagc aaatggtttg ac 22
<210> 38
   <211> 20
   <212> DNA
   <213> Artificial Sequence
<220>
   <223> Primer
<400> 38
   tatcttgcac ctggataacc 20
<210> 39
   <211> 59
   <212> DNA
   <213> Artificial Sequence
<220>
   <223> Primer
<400> 39
   caaaaagtta acatgcatca ccatcaccat cacaatctaa gaggtaaagt tcaacattc 59
<210> 40
   <211> 55
   <212> DNA
   <213> Artificial Sequence
<220>
   <223> Primer
<400> 40
   gttatctgca ggttacccag tggtacgaag cgccattggt ttgccgtgtg gattg 55
<210> 41
   <211> 56
   <212> DNA
   <213> Artificial Sequence
<220>
   <223> Primer
<400> 41
   ctgccggtct ccctatagtg agtcgtatta atttcggagt tcaacaaccg ttcaag 56
<210> 42
   <211> 54
   <212> DNA
   <213> Artificial Sequence
<220>
   <223> Primer
<400> 42
   taactaatta catgactcga ggtcgacggt atcatgaagt tgatgctgct ttgg 54
<210> 43
   <211> 25
   <212> DNA
   <213> Artificial Sequence
<220>
   <223> Primer
<400> 43
   atttagaagc tagaggttca gaaag 25
<210> 44
   <211> 24
   <212> DNA
   <213> Artificial Sequence
<220>
   <223> Primer
<400> 44
   tagaagaatg accatgccat atag 24
<210> 45
   <211> 73
   <212> DNA
   <213> Artificial Sequence
<220>
   <223> Primer
<400> 45
   ttttaatttt aatcaaaaag ttaacatgca tcaccatcac catcacactc acagagtcaa 60
   ctcctgtata ttc 73
<210> 46
   <211> 75
   <212> DNA
   <213> Artificial Sequence
<220>
   <223> Primer
<400> 46
   tgaatgtaag cgtgacataa ctaattacat gactcgaggt cgacggtatc tctggcggta 60
   ttgaactttg tggag 75
<210> 47
   <211> 60
   <212> DNA
   <213> Artificial Sequence
<220>
   <223> Primer
<400> 47
   gttatctgca ggttacccag tggtaaagtg tatggatggg ttgaagtatg tctttatatc 60
<210> 48
   <211> 58
   <212> DNA
   <213> Artificial Sequence
<220>
   <223> Primer
<400> 48
   acgaagttat gagctcgaat tcatcgatgc tacccggtgc tgcaaagact ttactaag 58
<210> 49
   <211> 24
   <212> DNA
   <213> Artificial Sequence
<220>
   <223> Primer
<400> 49
   gtgaatggtt aatagtgcgc tatg 24
<210> 50
   <211> 25
   <212> DNA
   <213> Artificial Sequence
<220>
   <223> Primer
<400> 50
   ctaacaaata ccacttcgac atcag 25
<210> 51
   <211> 73
   <212> DNA
   <213> Artificial Sequence
<220>
   <223> Primer
<400> 51
   ttttaatttt aatcaaaaag ttaacatgca tcaccatcac catcacacct tccgtgagat 60
   ttcccttgtt tac 73
<210> 52
   <211> 58
   <212> DNA
   <213> Artificial Sequence
<220>
   <223> Primer
<400> 52
   tatacgaagt tatctgcagg ttacccagtg gtataaccca taaccagtga tgttaacc 58
<210> 53
   <211> 47
   <212> DNA
   <213> Artificial Sequence
<220>
   <223> Primer
<400> 53
   gaagttatga gctcgaattc atcgatgacc actggtgttg ttgatcg 47
<210> 54
   <211> 75
   <212> DNA
   <213> Artificial Sequence
<220>
   <223> Primer
<400> 54
   tgaatgtaag cgtgacataa ctaattacat gactcgaggt cgacggtatc cgacggtaat 60
   gaggatgtaa atgag 75
<210> 55
   <211> 25
   <212> DNA
   <213> Artificial Sequence
<220>
   <223> Primer
<400> 55
   aaacaagagc agcatgcaac ttgag 25
<210> 56
   <211> 22
   <212> DNA
   <213> Artificial Sequence
<220>
   <223> Primer
<400> 56
   agtgacacca ggaactctaa ag 22
<210> 57
   <211> 57
   <212> DNA
   <213> Artificial Sequence
<220>
   <223> Primer
<400> 57
   gctttacact ttatgcttcc ggctcctatg ttgaactatg tcaatatcga tcgtatg 57
<210> 58
   <211> 56
   <212> DNA
   <213> Artificial Sequence
<220>
   <223> Primer
<400> 58
   tatctgcagg ttacccagtg gtacgaagcg ccaaacagaa attggttcat gtgttg 56
<210> 59
   <211> 56
   <212> DNA
   <213> Artificial Sequence
<220>
   <223> Primer
<400> 59
   gccggtctcc ctatagtgag tcgtattaat ttctggtgta ccaatttggt tatttc 56
<210> 60
   <211> 58
   <212> DNA
   <213> Artificial Sequence
<220>
   <223> Primer
<400> 60
   atatcagtta ttaccctatg cggtgtgaaa tacacaagta caacaacaac agatttag 58
<210> 61
   <211> 22
   <212> DNA
   <213> Artificial Sequence
<220>
   <223> Primer
<400> 61
   tacccacctt tgacataatc ag 22
<210> 62
   <211> 22
   <212> DNA
   <213> Artificial Sequence
<220>
   <223> Primer
<400> 62
   attcaaatgg cgtaccttta ac 22
<210> 63
   <211> 55
   <212> DNA
   <213> Artificial Sequence
<220>
   <223> Primer
<400> 63
   gctttacact ttatgcttcc ggctcctatg ttgggactgc tacactccaa atatg 55
<210> 64
   <211> 59
   <212> DNA
   <213> Artificial Sequence
<220>
   <223> Primer
<400> 64
   ttatctgcag gttacccagt ggtacgaagc gccataatag aagaaacacg tcaaatacc 59
<210> 65
   <211> 54
   <212> DNA
   <213> Artificial Sequence
<220>
   <223> Primer
<400> 65
   gccggtctcc ctatagtgag tcgtattaat ttccagatca aaccacatca tgag 54
<210> 66
   <211> 41
   <212> DNA
   <213> Artificial Sequence
<220>
   <223> Primer
<400> 66
   gtagcagtga cgttcattgt gtaatgtgta tatgttttat c 41
<210> 67
   <211> 37
   <212> DNA
   <213> Artificial Sequence
<220>
   <223> Primer
<400> 67
   catatacaca ttacacaatg aacgtcactg ctacaac 37
<210> 68
   <211> 53
   <212> DNA
   <213> Artificial Sequence
<220>
   <223> Primer
<400> 68
   atatcagtta ttaccctatg cggtgtgaaa tacacaagca ccaacaccat tac 53
<210> 69
   <211> 17
   <212> DNA
   <213> Artificial Sequence
<220>
   <223> Primer
<400> 69
   gttgtgcgtg gcttgac 17
<210> 70
   <211> 22
   <212> DNA
   <213> Artificial Sequence
<220>
   <223> Primer
<400> 70
   ataatacagc accaccaact tc 22
<210> 71
   <211> 55
   <212> DNA
   <213> Artificial Sequence
<220>
   <223> Primer
<400> 71
   gctttacact ttatgcttcc ggctcctatg ttgggccatg agatgacttt gtacg 55
<210> 72
   <211> 57
   <212> DNA
   <213> Artificial Sequence
<220>
   <223> Primer
<400> 72
   ttatctgcag gttacccagt ggtacgaagc gccagttctt gtttgaattc gcgtttg 57
<210> 73
   <211> 55
   <212> DNA
   <213> Artificial Sequence
<220>
   <223> Primer
<400> 73
   gttatgagct cgaattcatc gatgatatca gggaccgtta attaccaaca atctc 55
<210> 74
   <211> 25
   <212> DNA
   <213> Artificial Sequence
<220>
   <223> Primer
<400> 74
   tgttaattaa ttatttgttt gtttg 25
<210> 75
   <211> 55
   <212> DNA
   <213> Artificial Sequence
<220>
   <223> Primer
<400> 75
   acaaacaaac aaacaaataa ttaattaaca atgtcattgg taatacctca aatag 55
<210> 76
   <211> 53
   <212> DNA
   <213> Artificial Sequence
<220>
   <223> Primer
<400> 76
   atatcagtta ttaccctatg cggtgtgaaa tacaaagcgg cttgagtaca tgc 53
<210> 77
   <211> 21
   <212> DNA
   <213> Artificial Sequence
<220>
   <223> Primer
<400> 77
   aactgacgtt tcaagaacat c 21
<210> 78
   <211> 24
   <212> DNA
   <213> Artificial Sequence
<220>
   <223> Primer
<400> 78
   ataaacttgc atttgttgca tacc 24
<210> 79
   <211> 56
   <212> DNA
   <213> Artificial Sequence
<220>
   <223> Primer
<400> 79
   gctttacact ttatgcttcc ggctcctatg ttgaaagtgt aaatagacgt catgag 56
<210> 80
   <211> 58
   <212> DNA
   <213> Artificial Sequence
<220>
   <223> Primer
<400> 80
   ttatctgcag gttacccagt ggtacgaagc gccactgtgt actaaacgtg ataaatcc 58
<210> 81
   <211> 55
   <212> DNA
   <213> Artificial Sequence
<220>
   <223> Primer
<400> 81
   gttatgagct cgaattcatc gatgatatca gggaccgtta attaccaaca atctc 55
<210> 82
   <211> 25
   <212> DNA
   <213> Artificial Sequence
<220>
   <223> Primer
<400> 82
   tgttaattaa ttatttgttt gtttg 25
<210> 83
   <211> 54
   <212> DNA
   <213> Artificial Sequence
<220>
   <223> Primer
<400> 83
   aaaacaaaca aacaaacaaa taattaatta acaatgagct ctcatcagtt tttg 54
<210> 84
   <211> 54
   <212> DNA
   <213> Artificial Sequence
<220>
   <223> Primer
<400> 84
   atatcagtta ttaccctatg cggtgtgaaa tacaagacga tgatgtcttg aatg 54
<210> 85
   <211> 21
   <212> DNA
   <213> Artificial Sequence
<220>
   <223> Primer
<400> 85
   aactgacgtt tcaagaacat c 21
<210> 86
   <211> 21
   <212> DNA
   <213> Artificial Sequence
<220>
   <223> Primer
<400> 86
   agtaacaatt gcagcaatac c 21

## Beschreibung

### Gebiet der Erfindung

Gegenstand der Erfindung sind gentechnisch modifizierte *Pichia ciferrii* Zellen, deren Verwendung sowie ein Verfahren zur Herstellung von Sphingoidbasen und Sphingolipiden.

### Stand der Technik

*Pichia ciferrii* wird bereits seit Anfang der 60er Jahre zur Herstellung von Sphingoidbasen und Sphingolipiden verwendet, vgl. Wickerham et al. 1960, J Bacteriol. 80, 484-91.

Die Ausbeuten der Wildtypstämme an Sphingoidbasen und Sphingolipiden ist stets verbesserungswürdig.

Aufgabe der Erfindung war es, *Pichia ciferrii* Zellen zur Verfügung zu stellen, die eine erhöhte Produktivität an Sphingoidbasen und Sphingolipiden aufweisen.

### Beschreibung der Erfindung

Überraschenderweise wurde gefunden, dass die im Folgenden beschriebenen Zellen mit reduzierten, spezifischen Enzymaktivitäten die der Erfindung gestellte Aufgabe zu lösen vermögen.

Gegenstand der vorliegenden Erfindung sind daher gentechnisch modifizierte *Pichia ciferrii* Zellen mit im Vergleich zu Ihrem Wildtyp verminderten Aktivitäten der Enzyme wie im vorliegenden Anspruch 1 beschrieben.
Ein weiterer Gegenstand der Erfindung ist die Verwendung der vorgenannten Zellen sowie ein Verfahren zur Herstellung von Sphingoidbasen und Sphingolipiden.

Ein Vorteil der vorliegenden Erfindung ist es, dass sich die erfindungsgemäßen Zellen zu hohen Zelldichten anziehen lassen.

Noch ein Vorteil der vorliegenden Erfindung ist es, dass die Zellen bei Anzucht in geeigneten Nährmedien deutlich erhöhte Titer an acetylierten Sphingoidbasen produzieren.

Ein weiterer Vorteil der vorliegenden Erfindung ist die hohe genetische Stabilität der Stämme, welche eine Reversion zum ursprünglichen Genotyp ausschließt. Die hohe genetische Stabilität erlaubt außerdem die Kultivierung in Abwesenheit von Antibiotika, da kein Selektionsdruck aufrechterhalten werden muss.

Ein weiterer Vorteil der vorliegenden Erfindung ist es, dass die Zellen zur biotechnologischen, umweltschonenden Produktion von Sphingoidbasen aus kostengünstigen und nachwachsenden Rohstoffen eingesetzt werden können.

Gegenstand der vorliegenden Erfindung ist eine *Pichia ciferrii* Zelle, welche dadurch gekennzeichnet ist, dass die Zelle im Vergleich zu Ihrem Wildtyp eine verminderte Aktivität mindestens eines der Enzyme aufweist,
welche kodiert werden durch die intronfreien Nukleinsäuresequenzen ausgewählt aus den beiden Gruppen A) und B) bestehend aus
A) Seq ID Nr. 1, Seq ID Nr. 3, Seq ID Nr. 5, Seq ID Nr. 7, Seq ID Nr. 9, Seq ID Nr. 11,
B) eine Sequenz, die zu mindestens 80 %, besonders bevorzugt zu mindestens 90 %, darüber hinaus bevorzugt zu mindestens 95 % und am meisten bevorzugt zu mindestens 99 % identisch zu einer der Sequenzen Seq ID Nr. 1, Seq ID Nr. 3, Seq ID Nr. 5, Seq ID Nr. 7, Seq ID Nr. 9, Seq ID Nr. 11 ist,
dadurch gekennzeichnet, dass die verminderte Aktivität mindestens eines der Enzyme eine Kombination verminderter Aktivitäten der Enzyme ausgewählt aus der Gruppe: Seq ID Nr. 3 oder deren Gruppe B Analoge;
Seq ID Nr. 5 oder deren Gruppe B Analoge;
Seq ID Nr. 7 oder deren Gruppe B Analoge;
Seq ID Nr. 9 oder deren Gruppe B Analoge;
Seq ID Nr. 11 oder deren Gruppe B Analoge;
Seq ID Nr. 1 oder deren Gruppe B Analoge und Seq ID Nr. 3 oder deren Gruppe B Analoge;
Seq ID Nr. 1 oder deren Gruppe B Analoge und Seq ID Nr. 5 oder deren Gruppe B Analoge;
Seq ID Nr. 3 oder deren Gruppe B Analoge und Seq ID Nr. 5 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 11 oder deren Gruppe B Analog und
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge und Seq ID Nr. 11 oder deren Gruppe B Analoge,
darstellt.

In diesem Zusammenhang erfindungsgemäß bevorzugte Nukleinsäuresequenzgruppe ist Gruppe A).

Unter einem "Wildtyp" einer Zelle wird im Zusammenhang mit der vorliegenden Erfindung bevorzugt der Ausgangsstamm verstanden, aus dem die erfindungsgemäße Zelle durch Manipulation an den Elementen (wie beispielsweise die Gene umfassend die genannten Nukleinsäuresequenzen kodierend für entsprechende Enzyme oder die in entsprechenden Genen enthaltenen Promotoren, die mit den genannten Nukleinsäuresequenzen funktional verknüpft sind), die die Aktivitäten der Enzyme kodiert durch die genannten Nukleinsäure Seq ID Nr. beeinflussen, hervorgegangen ist.

Unter dem Begriff "Aktivität eines Enzyms" im Zusammenhang mit dem durch Seq ID Nr. 1 oder 3 oder durch eine zu Seq ID Nr. 1 oder 3 zu mindestens 80 %, besonders bevorzugt zu mindestens 90 %, darüber hinaus bevorzugt zu mindestens 95 % und am meisten bevorzugt zu mindestens 99 % identische Sequenz kodierten Enzym ist immer die enzymatische Aktivität zu verstehen, die die Umsetzung 5,10-Methylentetrahydrofolat + L-Glycin + H₂O <=> Tetrahydrofolat + L-Serin katalysiert. Diese Aktivität wird bevorzugt bestimmt nach dem in Schlüpen, 2003 beschriebenen Verfahren.

Unter dem Begriff "Aktivität eines Enzyms" im Zusammenhang mit dem durch Seq ID Nr. 5 oder durch ein zu Seq ID Nr. 5 zu mindestens 80 %, besonders bevorzugt zu mindestens 90 %, darüber hinaus bevorzugt zu mindestens 95 % und am meisten bevorzugt zu mindestens 99 % identische Sequenz kodierten Enzym ist immer die enzymatische Aktivität zu verstehen, die die Umsetzung L-Serin <=> Pyruvat + NH₃ katalysiert.
Diese Aktivität wird bevorzugt bestimmt nach dem in Ramos and Wiame, Eur J Biochem. 1982 Apr;123(3):571-6 beschriebenen Verfahren

Unter dem Begriff "Aktivität eines Enzyms" im Zusammenhang mit dem durch Seq ID Nr. 7 oder durch ein zu Seq ID Nr. 7 zu mindestens 80 %, besonders bevorzugt zu mindestens 90 %, darüber hinaus bevorzugt zu mindestens 95 % und am meisten bevorzugt zu mindestens 99 % identische Sequenz kodierten Enzym ist immer die enzymatische Aktivität zu verstehen, die die Umsetzung ATP + Sphinganin <=> ADP + Sphinganin 1-phosphat katalysiert.
Diese Aktivität wird bevorzugt bestimmt nach dem in Lanterman and Saba, Biochem J. 1998 Jun 1;332 ( Pt 2):525-31 beschriebenen Verfahren.

Unter dem Begriff "Aktivität eines Enzyms" im Zusammenhang mit dem durch Seq ID Nr. 9 oder durch ein zu Seq ID Nr. 9 zu mindestens 80 %, besonders bevorzugt zu mindestens 90 %, darüber hinaus bevorzugt zu mindestens 95 % und am meisten bevorzugt zu mindestens 99 % identische Sequenz kodierten Enzym ist immer die enzymatische Aktivität zu verstehen, die die Umsetzung Sphinganin 1-Phosphat <=> Phosphoethanolamin + Palmitaldehyd katalysiert.
Diese Aktivität wird bevorzugt bestimmt nach dem in Van Veldhoven and Mannaerts, J Biol Chem. 1991 Jul 5;266(19):12502-7 beschriebenen Verfahren.

Unter dem Begriff "Aktivität eines Enzyms" im Zusammenhang mit dem durch Seq ID Nr. 11 oder durch ein zu Seq ID Nr. 11 zu mindestens 80 %, besonders bevorzugt zu mindestens 90 %, darüber hinaus bevorzugt zu mindestens 95 % und am meisten bevorzugt zu mindestens 99 % identische Sequenz kodierten Enzym ist die Höhe der Expressionsrate des betrachteten Enzyms, insbesondere die intrazelluläre Konzentration, zu verstehen. Bestimmt wird diese über unten beschriebene 2-D-Gel Technik oder Western-Blot-Verfahren.

Unter der Formulierung "im Vergleich zu ihrem Wildtyp verminderte Aktivität" wird vorzugsweise eine um mindestens 50% verminderte, besonders bevorzugt um mindestens 90%, darüber hinaus bevorzugt um mindestens 99.9%, darüber hinaus noch mehr bevorzugt um mindestens 99,99% und am meisten bevorzugt um mindestens 99,999% verminderte Aktivität bezogen auf die Wildtyp-Aktivität verstanden.
Die Verminderung der jeweiligen Aktivitäten der erfindungsgemäßen Zelle im Vergleich zu ihrem Wildtyp wird bestimmt nach oben beschriebenen Verfahren zur Bestimmung der Aktivität unter Einsatz von möglichst gleichen Zellzahlen/-konzentrationen, wobei die Zellen unter gleichen Bedingungen wie beispielsweise Medium, Begasung, Agitation angezogen wurden.
Die "Nukleotid-Identität" relativ zu den angebenden Sequenzen, kann mit Hilfe bekannter Verfahren bestimmt werden. Generell werden besondere Computerprogramme mit Algorithmen unter Berücksichtigung spezieller Erfordernisse verwendet.
Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
- GAP (Deveroy, J. et al., Nucleic Acid Research 12 (1984), Seite 387, Genetics Computer Group University of Wisconsin, Medicine (Wi), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der bekannte Smith-Waterman Algorithmus kann ebenso zur Bestimmung der Nukleotid-Identität verwendet werden.

Bevorzugte Parameter für die Bestimmung der "Nukleotid-Identität" sind bei Verwendung des BLASTN-Programms (Altschul, S. et al., Journal of Molecular Biology 215 (1990), Seiten 403-410:

| | |
|---|---|
| Expect Threshold: | 10 |
| Word size: | 28 |
| Match Score: | 1 |
| Mismatch Score: | -2 |
| Gap costs: | Linear |

Die vorstehenden Parameter sind die default-Parameter im Nukleotidsequenzvergleich.
Das GAP-Programm ist ebenso zur Verwendung mit den vorstehenden Parametern geeignet.
Eine Identität von 80 % gemäß dem vorstehenden Algorithmus bedeutet im Zusammenhang mit der vorliegenden Erfindung 80 % Identität. Das gleiche gilt für höhere Identitäten.
Durch den Begriff "welche kodiert werden durch die intronfreien Nukleinsäuresequenzen" ist klar herausgestellt, dass bei einem Sequenzvergleich mit den hier angegebenen Sequenzen die zu vergleichenden Nukleinsäuresequenzen zuvor um etwaige Introns bereinigt werden müssen.
Alle angegebenen Prozent (%) sind, wenn nicht anders angegeben, Massenprozent. Erfindungsgemäß bevorzugte Zellen sind dadurch gekennzeichnet, dass die Verminderung der enzymatischen Aktivität erreicht wird durch Modifikation mindestens eines Genes umfassend eine der Sequenzen ausgewählt aus den zuvor genannten Nukleinsäuresequenzgruppen A) und B), wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz und Aussetzen des Gens unter den Einfluss von RNA-Interferenz oder Austausch von Teilen des Gens mit Fremd-DNA, insbesondere des Promoterbereichs.
Unter Fremd-DNA ist in diesem Zusammenhang jegliche DNA-Sequenz zu verstehen, die dem Gen (und nicht dem Organismus) "fremd" ist, d.h. auch *Pichia ciferrii* endogene DNA-Sequenzen können in diesem Zusammenhang als "Fremd-DNA" fungieren.
In diesem Zusammenhang ist es insbesondere bevorzugt, dass das Gen durch Insertion eines Selektionsmarkergens unterbrochen wird, somit die Fremd-DNA ein Selektionsmarkergen ist, insbesondere eines umfassend eine für das nat1 Gen aus *Streptomyces noursei* kodierende Sequenz, welche bevorzugt flankiert ist durch die Sequenz des *Pichia* PDA1-Promotors und die Sequenz des *Pichia* TEF-Terminators, wie beispielsweise beschrieben in Schorsch et al., 2009; Current Genetics (2009), 55(4), 381-389, wobei die für das nat1 Gen aus *Streptomyces noursei* kodierende Sequenz bevorzugt für *P. ciferrii* kodon optimiert ist, wobei bevorzugt die Insertion durch homologe Rekombination in den Genlocus erfolgte.
In diesem Zusammenhang kann es vorteilhaft sein, wenn das Selektionsmarkergen durch weitere Funktionalitäten erweitert wird, die wiederum eine anschließende Entfernung aus dem Gen ermöglichen, dies kann beispielsweise durch dem Organismus fremde Rekombinationssysteme, wie etwa ein Cre/loxP-System oder FRT (*Flippase Recognition Target*)-*System* oder das dem Organismus eigene homologe Rekombinationssystem erreicht werden.

In dem Zusammenhang mit den oben gelisteten Kombinationen ist es bevorzugt, die durch die Mitglieder der Gruppe A kodierten Enzymaktivitäten zu vermindern.

Erfindungsgemäß bevorzugte Zellen sind dadurch gekennzeichnet, dass die *Pichia ciferrii* Zelle sich ableitet von Stämmen ausgewählt aus der Gruppe bestehend aus *Pichia ciferrii NRRL Y-1031 F-60-10*, und dem Stamm *Pichia ciferri* CS.PCΔPro2, beschrieben in Schorsch et al., 2009, Curr Genet. 55, 381-9.

Erfindungsgemäß bevorzugte Zellen sind dadurch gekennzeichnet, dass die Zelle verglichen zu ihrem Wildtyp eine erhöhte enzymatische Aktivität mindestens eines der Enzyme ausgewählt aus
einem Enzym E₁, welches die Reaktion von Serin und Palmitoyl-CoA zu 3-Ketosphinganin katalysiert, insbesondere einer Serin-Palmitoyltransferase, insbesondere solche kodiert durch Seq ID Nr. 13 und /oder Seq ID Nr. 15,
einem Enzym E₂, welches die Reaktion von Sphinganin zu Phytosphingosin katalysiert, insbesondere einer Sphinganin C4-Hydroxylase, insbesondere solcher kodiert durch Seq ID Nr. 17.
Unter dem Begriff "Aktivität eines Enzyms" im Zusammenhang mit dem Enzym E₁ ist immer die enzymatische Aktivität zu verstehen, die die Umsetzungen von Palmitoyl-CoA + L-Serine <=> CoA + 3-Dehydro-D-Sphinganine + CO₂ katalysiert.
Diese Aktivität wird bevorzugt bestimmt nach dem in Zweerink et al., J Biol Chem. 1992 Dec 15;267(35):25032-8 beschriebenen Verfahren.

Unter dem Begriff "Aktivität eines Enzyms" im Zusammenhang mit dem Enzym E₂ ist immer die enzymatische Aktivität zu verstehen, die Umsetzung Sphinganin + NADPH + H⁺ + O₂ <=> Phytosphingosine + NADP⁺ + H₂O katalysiert.

Diese Aktivität wird bevorzugt bestimmt nach dem in Grilley et al., J Biol Chem. 1998 May 1;273(18):11062-8 beschriebenen Verfahren.

Der Begriff "gesteigerte Aktivität eines Enzyms", wie er vorstehend und in den nachfolgenden Ausführungen im Zusammenhang mit der vorliegenden Erfindung verwendet wird, ist vorzugsweise als gesteigerte intrazelluläre Aktivität zu verstehen. Die nun folgenden Ausführungen zur Erhöhung der Enzymaktivität in Zellen gelten sowohl für die Erhöhung der Aktivität des Enzyms E₁ bis ₂ als auch für alle nachfolgend genannten Enzyme, deren Aktivität gegebenenfalls erhöht werden kann. Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder eine Kombination dieser Methoden mit einem Vektor erzeugt, der das gewünschte Gen, ein Allel dieses Gens oder Teile davon und einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt. Einen Überblick über die Möglichkeiten zur Erhöhung der Enzym-Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar.
Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optischer Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden.

Es ist erfindungsgemäß bevorzugt, dass die Zelle, die verglichen zu ihrem Wildtyp eine erhöhte enzymatische Aktivität des Enzyms E₁ aufweist, im Vergleich zu Ihrem Wildtyp eine Kombination verminderter Aktivitäten der Enzyme aufweist, welche kodiert werden durch die intronfreien Nukleinsäuresequenzen:
in der Kombination Seq ID Nr. 1 oder deren Gruppe B Analoge und Seq ID Nr. 3 oder
deren Gruppe B Analoge und Seq ID Nr. 5 oder deren Gruppe B Analoge,
oder in der Kombination Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge und Seq ID Nr. 11 oder deren Gruppe B Analoge.

Es ist erfindungsgemäß bevorzugt, dass die Zelle, die verglichen zu ihrem Wildtyp eine erhöhte enzymatische Aktivität der Enzyme E₁ und E₂, aufweist, im Vergleich zu Ihrem Wildtyp eine Kombination verminderter Aktivitäten der Enzyme aufweist, welche kodiert werden durch die intronfreien Nukleinsäuresequenzen:
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge und Seq ID Nr. 11 oder deren Gruppe B Analoge.

In einer Ausführungsvariante stellen erfindungsgemäße *P. ciferri* Zellen, solche dar, wie sie in der WO2006048458 und WO2007131720 beschreiben sind und zusätzlich über die oben, im Zusammenhang mit den vorliegenden, erfindungsgemäßen Zellen beschriebenen enzymatischen Aktivitätsänderungen verfügen.

Ein weiteren Beitrag zur Lösung der der Erfindung gestellten Aufgabe leistet die Verwendung der erfindungsgemäßen Zellen zur Herstellung von Sphingoidbasen und Sphingolipiden.
Unter dem Begriff "Sphingoidbasen" im Zusammenhang mit der vorliegenden Erfindung sind Phytosphingosin, Sphingosin, Sphingadienin, 6-Hydroxysphingosin und Sphinganin (Dihydrosphingosin), auch in der acetylierten Form, wie z.B. Tetraacetylphytosphingosin, Triacetylphytosphingosin, Diacetylphytosphingosin, O-Acetylphytosphingosin, Triacetylsphinganin, Diacetylsphinganin, O-Acetylsphinganin, Triacetylsphingosin, Diacetylsphingosin, O-Acetylsphingosin, Tetraacetyl-6-Hydroxysphingosin, Triacetyl-6-Hydroxysphingosin, Diacetyl-6-Hydroxysphingosin, O-Acetyl-6-Hydroxysphingosin, Triacetylsphingadienin, Diacetylsphingadienin, O-Acetylsphingadienin zu verstehen.
Unter dem Begriff "Sphingolipide" im Zusammenhang mit der vorliegenden Erfindung sind Verbindungen zu verstehen, welche kovalent über eine Amidbindung mit einer Fettsäure verknüpfte Sphingoidbasen umfassen. Die Fettsäure kann gesättigt oder einfach bzw. mehrfach ungesättigt sein. Die Länge der Fettsäureseitenkette kann variieren. Die Fettsäureseitenkette kann ferner funktionelle Gruppen wie Hydroxygruppen aufweisen. Zu den Sphingolipiden zählen z.B. Phytoceramide, Ceramide und Dihydroceramide, sowie die komplexeren Glucosylceramide (Cerebroside) und die Inositolphosphorylceramide, Mannosyl-Inositolphosphorylceramide und Mannosyl-Di-Inositolphosphorylceramide. Zu den Sphingolipiden werden hier auch über eine Amidbindung mit einem Acetylrest verknüpfte Sphingoidbasen gezählt, wie z.B. N-Acetylphytosphingosin, N-Acetylsphinganin, N-Acetylsphingosin, N-Acetyl-6-Hydroxysphingosin. Diese Verbindungen sind auch unter dem Begriff Kurzkettenceramide bekannt.

Insbesondere die Verwendung der erfindungsgemäßen Zellen zur Herstellung von Sphingoidbasen und Sphingolipiden ausgewählt aus der Gruppe, Phytosphingosin, Sphingosin, Sphingadienin, 6-Hydroxysphingosin, Sphinganin (Dihydrosphingosin), Tetraacetylphytosphingosin (TAPS), Triacetylphytosphingosin, Diacetylphytosphingosin, O-Acetylphytosphingosin, N-Acetylphytosphingosin, Triacetylsphinganin (TriASa), Diacetylsphinganin, O-Acetylsphinganin, N-Acetylsphinganin, Triacetylsphingosin (TriASo), Diacetylsphingosin, O-Acetylsphingosin, N-Acetylsphingosin, Tetraacetyl-6-Hydroxysphingosin, Triacetyl-6-Hydroxysphingosin, Diacetyl-6-Hydroxysphingosin, O-Acetyl-6-Hydroxysphingosin, N-Acetyl-6-Hydroxysphingosin, Triacetylsphingadienin, Diacetylsphingadienin, O-Acetylsphingadienin ist vorteilhaft. Ganz besonders bevorzugt ist die Verwendung der erfindungsgemäßen Zellen zur Herstellung von Tetraacetylphytosphingosin (TAPS). Eine erfindungsgemäß bevorzugte Verwendung ist erfindungsgemäß dadurch gekennzeichnet, dass erfindungsgemäß bevorzugte, wie oben beschriebene Zellen verwendet werden.

*P. ciferri* Zellen welche insbesondere zu Herstellung von den oben beschriebenen Sphingsosin- und Sphinganin-Derivaten verwendet werden sind solche, wie sie in der WO2006048458 und WO2007131720 beschreiben sind und zusätzlich über die oben im Zusammenhang mit den vorliegenden, erfindungsgemäßen Zellen beschriebenen enzymatischen Aktivitätsänderungen verfügen.

Einen weiteren Beitrag zur Lösung der der Erfindung gestellten Aufgabe leistet ein Verfahren zur Herstellung der zuvor beschriebenen erfindungsgemäßen Zelle umfassend die Verfahrensschritte:
I) Bereitstellen einer *Pichia ciferri* Zelle und
II) Modifikation mindestens eines Genes umfassend eine der Sequenzen ausgewählt aus den in Anspruch 1 genannten Nukleinsäuresequenzgruppen A) und B) durch
   Insertion von Fremd-DNA, insbesondere von DNA kodierend für ein Selektionsmarkergen, bevorzugt eins, welches rückstandslos entfernt werden kann und eine Deletion im Ziel-Gen hinterlässt, in das Gen,
   Deletion mindestens von Teilen des Gens,
   Punktmutationen in der Gensequenz,
   Aussetzen des Gens unter den Einfluss von RNA-Interferenz und Austausch von Teilen des Gens mit Fremd-DNA, insbesondere des Promoterbereichs.

Einen weiteren Beitrag zur Lösung der der Erfindung gestellten Aufgabe leistet ein Verfahren zur Herstellung von Sphingoidbasen und Sphingolipide umfassend die Verfahrensschritte
a) In Kontakt bringen der erfindungsgemäßen Zelle mit einem Medium beinhaltend eine Kohlenstoffquelle,
b) Kultivieren der Zelle unter Bedingungen, die es der Zelle ermöglichen aus der Kohlenstoffquelle Sphingoidbasen und Sphingolipide zu bilden und
c) gegebenenfalls Isolierung der gebildeten Sphingoidbasen und Sphingolipide. Erfindungsgemäß bevorzugte Verfahren setzen oben als erfindungsgemäß bevorzugt genannte Zellen ein.
Als Kohlenstoffquelle können Kohlehydrate wie z. B. Glukose, Fruktose, Glycerin, Saccharose, Maltose, Melasse, aber auch Alkohole wie z.B. Ethanol sowie organische Säuren wie z.B. Acetat eingesetzt werden. Als Stickstoffquelle können z.B. Ammoniak, Ammoniumsulfat, Ammoniumnitrat, Ammoniumchlorid, organische Stickstoffverbindungen (wie Hefeextrakt, Malzextrakt, Pepton, Maisquellwasser) eingesetzt werden. Des Weiteren können anorganische Verbindungen wie z.B. Phosphat-, Magnesium-, Kalium-, Zink-, Eisensalze und andere eingesetzt werden. Geeignete Kultivierungsbedingungen für *Pichia ciferri* sind dem Fachmann beispielsweise aus der WO2006048458 und der WO2007131720 bekannt.
Das erfindungsgemäße Verfahren ist insbesondere zur Herstellung von Tetraacetylphytosphingosine (TAPS) geeignet.
In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Folgende Abbildungen sind Bestandteil der Beispiele:
Abbildung 1: Konstruktionsprinzip der Gendeletionskassetten
Abbildung 2: Konstruktionsprinzip der Überexpressionskassetten

### Beispiele:

### Konstruktion von Gendeletionskassetten

Gendeletionen wurden, wenn nicht anders angegeben, mittels klassischem "one-step gene replacement" durchgeführt wie in Rothstein 1983, Methods Enzymol 101: 202-211 beschrieben
Deletionskassetten wurden über *in vivo* Klonierung konstruiert, wodurch schlussendlich Plasmide erhalten wurden, die als Matrize zur PCR basierten Amplifikation der Deletionskassetten eingesetzt wurden. Diese PCR Produkte wurden anschließend in *P. ciferrii* transformiert, mit dem Ziel ein bestimmtes Gen zu deletieren.
Für die Konstruktion der Deletionskassetten wurde das Plasmid p426HXT7-6HIS (Hamacher et al., 2000; Microbiology 148, 2783-8) als Shuttle-Vektor eingesetzt. p426HXT7-6HIS wurde zunächst mit BamHI und EcoRI geschnitten, wodurch ein 5,69 kb Fragment erhalten wurde, das als Grundgerüst für die nachfolgenden Klonierungen eingesetzt wurde. Für jede *P*. *ciferrii*-Deletionskassette wurden zunächst per PCR drei überlappende DNA-Fragmente generiert: Als Mittelteil ein später wieder eliminierbarer dominanter clonNAT-Marker (*nat1*-Resistenzkassette) (vgl. Schorsch et al., Curr Genet. 2009 Aug;55(4):381-9), ein zweites Fragment von etwa 500 bp Länge repräsentierend die 5'-untranslatierte Region des zu deletierenden ORFs (Promoter-Region, PR) und mit Überlappung zum Beginn des clonNAT-Marker-Fragments, sowie ein drittes Fragment von etwa 500 bp Länge repräsentierend die 3'-untranslatierte Region (Terminator-Region, TR) des zu deletierenden ORFs und mit Überlappung zum Ende des clonNAT-Marker-Fragments.
Für die Konstruktion jeder Deletionskassette wurden die Promoter- (PR) und die
Terminator-Region (TR) des zu deletierenden Gens aus genomischer *P. ciferrii* Wildtyp DNA mittels PCR amplifiziert, wobei jeweils Gen-spezifische Primer eingesetzt wurden. Hierzu wurden jeweils Primerpaare P1/P2 für die PR und P3/P4 für die TR verwendet. Die Primer wurden so gewählt, dass diese am 5' Ende etwa 30-35 bps lange Bereiche besaßen, die überlappend mit den zu fusionierenden DNA Elementen waren:

| **Primer** | **Überlappung des 5'-Endes mit:** |
|---|---|
| P1 | Klonierungsvektor p426HXT7-6HIS |
| P2 | *nat1*-Resistenzkassette (PCR-Amplifikat des Plasmids pCS.LoxP.nat1 mit den Primern LPNTL.fw und LPNTL.rv) |
| P3 | *nat1*-Resistenzkassette |
| P4 | Klonierungsvektor p426HXT7-6HIS |

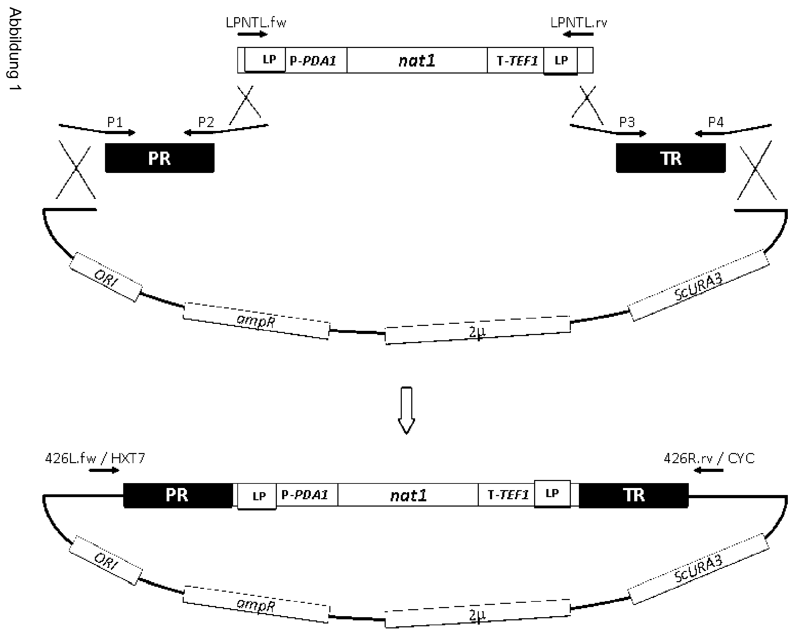

Für die Amplifizierung des mittleren Fragments (*nat1*-Resistenzkassette, Seq ID Nr. 19) wurde in jedem Fall das Primerpaar LPNTL.fw (TGGCGCTTCGTACCACTGGGTAAC) und LPNTL.rv (GAAATTAATACGACTCACTATAGG) eingesetzt, wobei das Plasmid pCS.LoxP.nat1 (Schorsch et al., 2009; Curr. Genet. 55, 381-9) als Matrize eingesetzt wurde (alle Primersequenzen sind in 5' → 3'-Orientierung angegeben).
Die PCR Produkte der Primerpaare P1/P2, P3/P4 und LPNTL.fw/LPNTL.rv wurden zusammen mit dem zuvor durch BamHI und EcoRI -Verdau linearisierten Plasmid p426HXT7-6HIS in den *S*. *cerevisiae* Stamm K26 transformiert. *In vivo* wurden die PCR Produkte mit dem linearisierten Vektor über homologe Rekombination zusammengefügt, wodurch der linearisierte Vektor rezirkularisiert wurde und in *S*. *cerevisiae* vermehrt werden konnte. Erhaltene Transformanten wurden mittels des Markergens (*nat1*) auf YEPD Platten mit clonNAT selektiert, deren DNA isoliert, in *E*. *coli* transformiert und die daraus reisolierten Plasmide durch Restriktionskartierung bzw. Sequenzierung verifiziert. Zur Amplifikation der Deletionskassetten wurden (wenn nicht anders erwähnt) die Primer-Paare 426L.fw (GCTTCCGGCTCCTATGTTG, Seq ID Nr. 23 ) und 426R.rv (ACCCTATGCGGTGTGAAATAC, Seq ID Nr. 24) oder HXT7 (GCCAATACTTCACAATGTTCGAATC, Seq ID Nr. 25) und CYC (CGTGAATGTAAGCGTGACATAAC, Seq ID Nr. 26) verwendet. Zur Verdeutlichung vergleiche Abbildung 1.

Für die sukzessive Deletion mehrerer Gene wurde nach jeder Deletion ein Marker-Rescue durchgeführt. Dies wurde durch Transformation mit Plasmid pCS.opt.Cre (Seq ID. No 20) bewerkstelligt wie zuvor beschrieben (Schorsch et al., Curr Genet. 2009 Aug;55(4):381-9). Verifizierungen der Gendeletionen wurden durch PCR-Analysen mit genomischer DNA der Transformanten als Matrize vorgenommen.

Die jeweiligen Gendeletionskassetten der Gene mit Sequenzen Seq ID Nr. 1, Seq ID Nr. 3, Seq ID Nr. 5, Seq ID Nr. 7, Seq ID Nr. 9 und Seq ID Nr. 11 wurden mit den Primern in unten stehender Tabelle konstruiert. Dabei wurden für jede der Seq IDs jeweils die ersten beiden aufgeführten Primer (SH11 und SH12 bzw. SH21 und SH22 bzw. C1 und C2 bzw. HXT7-LCB4.fw und LCB4.HXT7.rv bzw. HXT7-DPL1.fw und DPL1.rv2 bzw. ORM-426L.fw und ORM-LPNTL.rv) zur Amplifizierung der PR genutzt, die nächsten beiden aufgeführten Primer (SH13 und SH14 bzw. SH23 und SH24 bzw. C3 und C4 bzw. LCB4.rv und LCB4.fw bzw. DPL1.fw2 und CYC-DPL1.rv bzw. ORM-LPNTL.fw2 und ORM-426R.rv) zur Amplifizierung der TR. Die jeweils beiden letztgenannten Primer (SHMT1.pop-in.fw und SHMT1.veri.rv bzw. SHMT2.pop-in.fw und SHMT2.veri.rv bzw. CHA1.pop-in.fw und CHA1.veri.rv bzw. LCB4.pop-in.fw und LCB4.veri.rv bzw. DPL1.pop-in.fw und DPL1.veri.rv bzw. ORM1.pop-in.fw und ORM.veri.rv) dienen dem Integrationsnachweis bzw. dem Nachweis des Wildtypallels.

| **Gen** | **Primername** | **Sequenz (5' -> 3')** |
|---|---|---|
| | SH11 Seq ID Nr. 27 | |
| Seq ID Nr. 1 | SH12 Seq ID Nr. 28 | |
| | SH13 Seq ID Nr. 29 | |
| | SH14 Seq ID Nr. 30 | |
| | SHMT1.pop-in.fw Seq ID Nr. 31 | TTGATAGGGCAAATTCTCCAAC |
| | SHMT1.veri.rv | TTCACCTGGATAACCTTCTG |
| | Seq ID Nr. 32 | |
| | SH21 Seq ID Nr. 33 | |
| | SH22 Seq ID Nr. 34 | |
| Seq ID Nr. 3 | SH23 Seq ID Nr. 35 | |
| | SH24 Seq ID Nr. 36 | |
| | SHMT2.pop-in.fw Seq ID Nr. 37 | AAGTTTCAGCAAATGGTTTGAC |
| | SHMT2.veri.rv Seq ID Nr. 38 | TATCTTGCACCTGGATAACC |
| | C1 Seq ID Nr. 39 | |
| | C2 Seq ID Nr. 40 | |
| Seq ID Nr. 5 | C3 Seq ID Nr. 41 | |
| | C4 Seq ID Nr. 42 | |
| | CHA1.pop-in.fw Seq ID Nr. 43 | ATTTAGAAGCTAGAGGTTCAGAAAG |
| | CHA1.veri.rv Seq ID Nr. 44 | TAGAAGAATGACCATGCCATATAG |
| | HXT7-LCB4.fw Seq ID Nr. 45 | |
| | LCB4.HXT7.rv Seq ID Nr. 46 | |
| Seq ID Nr. 7 | LCB4.rv Seq ID Nr. 47 | |
| | LCB4.fw Seq ID Nr. 48 | |
| | LCB4.pop-in.fw Seq ID Nr. 49 | GTGAATGGTTAATAGTGCGCTATG |
| | LCB4.veri.rv Seq ID Nr. 50 | CTAACAAATACCACTTCGACATCAG |
| | HXT7-DPL1.fw Seq ID Nr. 51 | |
| | DPL1.rv2 Seq ID Nr. 52 | |
| Seq ID Nr. 9 | DPL1.fw2 Seq ID Nr. 53 | |
| | CYC-DPL1.rv Seq ID Nr. 54 | |
| | DPL1.pop-in.fw Seq ID Nr. 55 | AAACAAGAGCAGCATGCAACTTGAG |
| | DPL1.veri.rv Seq ID Nr. 56 | AGTGACACCAGGAACTCTAAAG |
| | ORM-426L.fw Seq ID Nr. 57 | |
| | ORM-LPNTL.rv Seq ID Nr. 58 | |
| Seq ID Nr. 11 | ORM-LPNTL.fw2 Seq ID Nr. 59 | |
| | ORM-426R.rv Seq ID Nr. 60 | |
| | ORM1.pop-in.fw Seq ID Nr. 61 | TACCCACCTTTGACATAATCAG |
| | ORM.veri.rv Seq ID Nr. 62 | ATTCAAATGGCGTACCTTTAAC |

### Konstruktion von Überexpressionskassetten

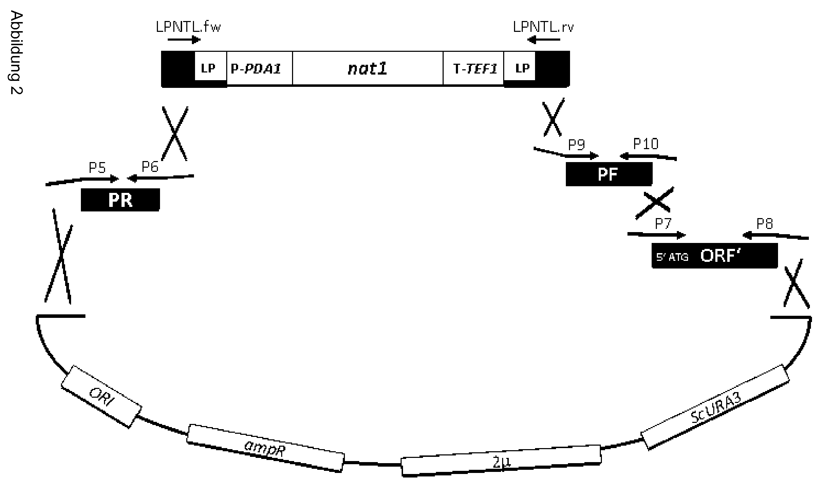

Die Konstruktion von Überexpressionskassetten erfolgte prinzipiell analog zu dem für die Deletionskassetten angewandten Verfahren. Allerdings wurde im Falle der Überexpressionskassetten ein zusätzliches viertes PCR-Produkt generiert (Promotor-Fragment, PF), welches ein Fragment des PcTDH3- oder des PcENO1-Promotors repräsentiert. Dieses wurde später *in vivo* mit der *nat1*-Resistenzkassette und dem dritten PCR-Fragment verknüpft, welches in diesem Fall eine Überlappung mit dem Beginn des überzuexprimierenden ORFs aufwies. (siehe Abb. 2).

Für eine Überexpression des Genprodukts Seq ID Nr. 13 wurde in *P. ciferrii* der native Promotor durch das *PcEANO1*⁻⁵⁸⁴⁻¹ (Seq ID Nr. 21) Promoter-Fragment ausgetauscht. Für eine Überexpression der Genprodukte Seq ID Nr. 15 und Seq ID Nr. 17 hingegen wurde in *P. ciferrii* der jeweilige native Promotor durch das *PcTDH3*⁻⁴²⁰⁻¹ Promoter-Fragment ausgetauscht (Seq ID Nr. 22).
Grundsätzlich wurden für die Konstruktion der jeweiligen Überexpressionskassetten 3 verschiedene Gen-spezifische Primer-Paare verwendet. Die Primer wurden so gewählt, dass diese am 5' Ende etwa 30-35 bps lange Bereiche besaßen, die überlappend mit den zu fusionierenden DNA Elementen waren.

| **Primer** | **Überlappung des 5'-Endes mit:** |
|---|---|
| P5 | Klonierungsvektor p426HXT7-6HIS |
| P6 | *nat1*-Resistenzkassette (PCR-Amplifikat des Plasmids pCS.LoxP.nat1 mit den Primern LPNTL.fw und LPNTL.rv) |
| P9 | *nat1*-Resistenzkassette |
| P10 | 5'-Ende des überzuexprimierenden ORFs |
| P7 | 3'-Ende des *PcENO1*⁻⁵¹⁴⁻¹ bzw. des *PcTDH3⁻⁴²⁰⁻¹* Promoter-Fragments |
| P8 | Klonierungsvektor p426HXT7-6HIS |

Für die Amplifizierung der *nat1*-Resistenzkassette wurde in jedem Fall das Primer-Paar LPNTL.fw und LPNTL.rv eingesetzt, wobei das Plasmid pCS.LoxP.nat1 (Schorsch et al., 2009; Curr. Genet. 55, 381-9) als Matrize eingesetzt wurde. Die PCR Produkte der Primerpaare P5/P6, P7/P8, P9/P10 und LPNTL.fw/LPNTL.rv wurden zusammen mit dem zuvor durch Hpal- und NgoMIV-Verdau linearisierten Plasmid p426HXT7-6HIS in den *S. cerevisiae* Stamm K26 transformiert. *In vivo* wurden die PCR Produkte mit dem linearisierten Vektor über homologe Rekombination zusammengefügt, wodurch der linearisierte Vektor rezirkularisiert wurde und in *S*. *cerevisiae* vermehrt werden konnte. Erhaltene Transformanden wurden mittels des Markergens (*nat1*) auf YEPD Platten mit clonNAT selektiert, deren DNA isoliert, in *E. coli* transformiert und die daraus reisolierten Plasmide durch Restriktionskartierung bzw. Sequenzierung verifiziert. Zur Amplifikation der Überexpressionskassetten wurde in jedem Fall das Primer-Paar "426L.fw & 426R.rv" verwendet.

### Zur Verdeutlichung vergleiche Abbildung 2.

Für die kombinierte Überexpression mehrerer Gene, bzw. für die Kombination von Überexpressionen eines oder mehrerer Ziel-Gene mit einer oder mehrerer Gendeletionen, wurde nach jedem Schritt (Deletion eines Ziel-Gens bzw. chromosomale Integration einer Überexpressionskassette) ein Marker-Rescue durchgeführt. Dies wurde durch Transformation mit Plasmid pCS.opt.Cre bewerkstelligt wie zuvor beschrieben (Schorsch et al., Curr Genet. 2009 Aug;55(4):381-9). Verifizierungen der Integration der Überexpressionskassetten wurden durch PCR-Analysen mit genomischer DNA der Transformanten als Matrize vorgenommen.

Die jeweiligen Überexpressionskassetten für Enzyme kodiert durch die Sequenzen Seq ID Nr. 13, Seq ID Nr. 15 und Seq ID Nr. 17 wurden mit den Primern in unten stehender Tabelle konstruiert. Dabei wurden für jede der Seq IDs jeweils die ersten beiden aufgeführten Primer (LCB1.426L.fw und LCB1.LPNTL.rv bzw. LCB2-426L.fw und LCB2-LPNTL.rv bzw. SYR2oe.426L und SYR2oe.LPNTL.rv) zur Amplifizierung der PR genutzt. Die nächsten beiden aufgeführten Primer (P-ENO.LPNTL.fw und LCB1.P-ENO.rv bzw. TDH3-LPNTL.fw und P-TDH3.rv bzw. TDH3-LPNTL.fw und P-TDH3.rv) wurden für die Amplifizierung des jeweiligen *PcENO1*⁻⁵⁸⁴⁻¹ bzw. *PcTDH3*⁻⁴²⁰⁻¹ Promoter-Fragments genutzt. Die nächsten beiden aufgeführten Primer (P-ENO.LCB1.fw und LCB1.426R.rv bzw. LCB2.P-TDH3.fw und LCB2-426R.rv bzw. SYR2oe.P-TDH3.fw und SYR2oe.426R) wurden zur Amplifizierung der 5'-ORF-Fragmente der jeweils überzuexprimierenden Ziel-Gene genutzt. Die jeweils beiden letztgenannte Primer (P-ENO.veri.rv und LCB1üe.veri.rv bzw. P-TDH3.pop.fw und LCB2üe.veri.rv bzw. P-TDH3.pop.fw und SYR2oe.veri.rv ) dienen dem Integrationsnachweis bzw. dem Nachweis des Wildtypallels

| **Gen** | **Primername** | **Sequenz (5' -> 3')** |
|---|---|---|
| Seq ID Nr. 13 | LCB1.426L.fw Seq ID Nr. 63 | |
| | LCB1.LPNTL.rv Seq ID Nr. 64 | |
| | P-ENO.LPNTL.fw Seq ID Nr. 65 | |
| | LCB1.P-ENO.rv Seq ID Nr. 66 | |
| | P-ENO.LCB1.fw Seq ID Nr. 67 | |
| | LCB1.426R.rv Seq ID Nr. 68 | |
| | P-ENO.veri.rv Seq ID Nr. 69 | GTTGTGCGTGGCTTGAC |
| | LCB1üe.veri.rv Seq ID Nr. 70 | ATAATACAGCACCACCAACTTC |
| | LCB2-426L.fw Seq ID Nr. 71 | |
| | LCB2-LPNTL.rv Seq ID Nr. 72 | |
| | TDH3-LPNTL.fw Seq ID Nr. 73 | |
| Seq ID Nr. 15 | P-TDH3.rv Seq ID Nr. 74 | TGTTAATTAATTATTTGTTTGTTTG |
| | LCB2.P-TDH3.fw Seq ID Nr. 75 | |
| | LCB2-426R.rv Seq ID Nr. 76 | |
| | P-TDH3.pop.fw Seq ID Nr. 77 | AACTGACGTTTCAAGAACATC |
| | LCB2üe.veri.rv Seq ID Nr. 78 | ATAAACTTGCATTTGTTGCATACC |
| Seq ID Nr. 17 | SYR2oe.426L Seq ID Nr. 79 | |
| | SYR2oe.LPNTL.rv Seq ID Nr. 80 | |
| | TDH3-LPNTL.fw Seq ID Nr. 81 | |
| | P-TDH3.rv Seq ID Nr. 82 | TGTTAATTAATTATTTGTTTGTTTG |
| | SYR2oe.P-TDH3.fw Seq ID Nr. 83 | |
| | SYR2oe.426R Seq ID Nr. 84 | |
| | P-TDH3 pop fw | AACTGACGTTTCAAGAACATC |
| | Seq ID Nr. 85 | |
| | SYR2oe.veri.rv Seq ID Nr. 86 | AGTAACAATTGCAGCAATACC |

### Produktion von acetylierten Sphingoidbasen mit den genetisch veränderten Stämmen

Erhöhte Titer acetylierter Sphingoidbasen wurden durch folgende genetische Modifikationen erreicht:

In den folgenden Tabellen werden die Titer acetylierter Sphingoidbasen (Tetraacetylphytosphingosin, TAPS und ggf. Triacetylsphinganin, TriASa) der verschiedenen rekombinanten *P. ciferrii-*Stämme nach Anzucht im Schüttelkolben bis zur Stationärphase gezeigt.
Details (verwendete Medien, Anzuchtbedingungen, Extraktion, Quantifizierung mittels HPLC-Analytik) sind in Schorsch et al., Curr Genet. 2009 Aug;55(4):381-9, beschrieben. Der vorliegend eingesetzte Stamm entspricht dem in der vorgenannten Literaturstelle bezeichneten *Pichia ciferri* CS.PCΔPro2, im Folgenden auch kurz "CS" bezeichnet.

Zunächst wurde der Einfluss von Deletionen verschiedener Gene auf die Produktion acetylierter Sphingoidbasen untersucht. Die Ergebnisse sind in der folgenden Tabelle gezeigt. Es zeigte sich, dass einzeln v.a. die Deletion von PcSHM2 zu einer deutlich gesteigerten Produktion acetylierter Sphingoidbasen führte. Dieser Effekt wurde noch verstärkt durch die Kombination mit einer PcSHM1-Deletion. Eine weitere Verstärkung wurde durch eine zusätzliche Deletion von PcCHA1 erzielt. Dieser Stamm mit dem relevanten Genotyp *cha1 shm1 shm2* ergab den mit Abstand höchsten Titer von 64 mg TAPS * g-1 (CDW) zzgl. 3 mg TriASa * g-1 (CDW).

Einfluss von Deletionen verschiedener Gene auf die Produktion acetylierter Sphingoidbasen:

| Stamm | relevanter Genotyp¹ | mg TAPS * g⁻¹ (CDW) | mg TriASa * g⁻¹ (CDW)² |
|---|---|---|---|
| CS | | 21 | |
| CS.S1 | *shm1* | 20 | |
| CS.S2 | *shm2* | 26 | |
| CS.SS | *shm1shm2* | 42 | |
| CS.C | *cha1* | 23 | |
| CS.CS1 | *cha1 shm1* | 23 | |
| CS.CS2 | *cha1 shm2* | 29 | |
| CS.CSS | *cha1 shm1 shm2* | 65 | 3 |

| | | | |
|---|---|---|---|
| Bezug zu den SEQ-IDs: *shm1,* SEQ-ID Nr. 1; *shm2,* SEQ-ID Nr. 3; *cha1,* SEQ-ID Nr. 5 ² Titer unterhalb 2 mg/g Zelltrockenmasse werden nicht gezeigt | | | |

Als nächstes wurde der Einfluss verschiedener genetischer Modifikationen zur Verstärkung von Enzymaktivitäten untersucht, und zwar im Hintergrund des Stammes CS.CSS (*cha1 shm1 shm2).* Dazu wurden, einzeln und in ausgewählten Kombinationen, folgende gentechnischen Modifikationen im Stamm CS.CSS vorgenommen:
Deletion von PcLCB4, Seq ID Nr. 7
Deletion von PcDPL1, Seq ID Nr. 9
Deletion von PcORM12, Seq ID Nr. 11
Überexpression von PcLCB1 Seq ID Nr. 13
Überexpression von PcLCB2 Seq ID Nr. 15
Überexpression von PcSYR2 Seq ID Nr. 17
Die Effekte der Deletionen von PcLCB4 bzw PcDPL1 wurden zudem auch für sich, also ohne Kombination mit dem cha1 shm1 shm2 Genotyp adressiert.

Um additive oder synergistische Effekte zu erzielen, wurden zahlreiche der für die Sphingoidbasenproduktion förderlichen genetischen Modifikationen in unterschiedlichen Kombinationen in einem Stamm vereint. Dabei erwies sich der Stamm mit folgendem Genotyp als am besten:
*cha1 shm1 shm2 Icb4 orm12 TDH3p:LCB2 ENO1p:LCB1 TDH3p:SYR2.*
Dieser Stamm lieferte im Schüttelkolben einen Titer von 199 mg TAPS * g⁻¹ (CDW) (zzgl. 12 mg Triacetyl-Sphinganin (TriASa) * g⁻¹ (CDW), während der Referenzstamm CS lediglich 21 mg TAPS * g⁻¹ (CDW) lieferte.

Die Ergebnisse sind in der folgenden Tabelle gezeigt.

Einfluss von gentechnischen Veränderungen des Sphingolipid-Stoffwechsels auf die Produktion acetylierter Sphingoidbasen

| Stamm | relevanter Genotyp^{1; 2} | mg TAPS * g⁻¹ (CDW) | mg TriASa * g⁻¹ (CDW)³ |
|---|---|---|---|
| CS | | 21 | |
| CS.L4 | *Icb4* | 54 | |
| CS.DPL1 | *dpl1* | 28 | |
| CS.S2 | *shm2* | 26 | |
| CS.SS | *shm1 shm2* | 42 | |
| CS.CSS | *cha1 shm1 shm2* | 65 | 3 |
| CSS.L1 | *cha1 shm1 shm2 NO1p:LCB1* | 74 | 2 |
| CSS.L2 | *cha1 shm1 shm2 TDH3p:LCB2* | 82 | 4 |
| CSS.L1.L2 | *cha1 shm1 shm2 ENO1p:LCB1 TDH3p:LCB2* | 102 | 8 |
| CSS.L4 | *cha1 shm1 shm2 Icb4* | 116 | 8 |
| CSS.O | *cha1 shm1 shm2 orm12* | 104 | 6 |
| CSS.D | *cha1 shm1 shm2 dpl1* | 84 | 3 |
| CSS.L4.O | *cha1 shm1 shm2 Icb4 orm12* | 172 | 8 |
| CSS.L4.O.L2 | *cha1 shm1 shm2 Icb4 orm12* | 182 | 16 |
| | *TDH3p:LCB2* | | |
| CSS.L4.O.L2.L1 | *cha1 shm1 shm2 Icb4 orm12* | 178 | 44 |
| | *TDH3p:LCB2* | | |
| | *ENO1p:LCB1* | | |
| CSS.L4.O.L2.L1.S2 | *cha1 shm1 shm2 Icb4 orm12* | 199 | 12 |
| | *TDH3p:LCB2* | | |
| | *ENO1p:LCB1* | | |
| | *TDH3p:SYR2* | | |

| | | | |
|---|---|---|---|
| ¹ Bezug zu den SEQ-IDs: *shm1,* SEQ-ID Nr. 1; *shm2,* SEQ-ID Nr. 3; *cha1,* SEQ-ID Nr. *Icb4,* SEQ-ID Nr. 7; *dpl1,* SEQ-ID Nr. 9; *orm12,* SEQ-ID Nr. 11; *LCB1*, SEQ-ID Nr. 13; *LCB2*, SEQ-ID Nr. 15; *SYR2*, SEQ-ID Nr. 17 ² Inaktivierte Gene sind in Kleinbuchstaben aufgeführt. Überexprimierte Gene sind in Grossbuchstaben und mit dem jeweiligen Promotor (Abkürzung "p") aufgeführt, unter dessen Kontrolle sie stehen. ³ Titer unterhalb 2 mg/g Zelltrockenmasse werden nicht gezeigt | | | |

## Patentansprüche

1. *Pichia ciferrii* Zelle, **dadurch gekennzeichnet, dass** die Zelle im Vergleich zu Ihrem Wildtyp eine verminderte Aktivität mindestens eines der Enzyme aufweist, welche kodiert werden durch die intronfreien Nukleinsäuresequenzen ausgewählt aus den beiden Gruppen A) und B) bestehend aus
A) Seq ID Nr. 1, Seq ID Nr. 3, Seq ID Nr. 5, Seq ID Nr. 7, Seq ID Nr. 9, Seq ID Nr. 11,
B) eine Sequenz, die zu mindestens 80 % identisch zu einer der Sequenzen Seq ID Nr. 1, Seq ID Nr. 3, Seq ID Nr. 5, Seq ID Nr. 7, Seq ID Nr. 9, Seq ID Nr. 11 ist,
**dadurch gekennzeichnet, dass** die verminderte Aktivität mindestens eines der Enzyme eine Kombination verminderter Aktivitäten der Enzyme ausgewählt aus der Gruppe:
Seq ID Nr. 3 oder deren Gruppe B Analoge;
Seq ID Nr. 5 oder deren Gruppe B Analoge;
Seq ID Nr. 7 oder deren Gruppe B Analoge;
Seq ID Nr. 9 oder deren Gruppe B Analoge;
Seq ID Nr. 11 oder deren Gruppe B Analoge;
Seq ID Nr. 1 oder deren Gruppe B Analoge und Seq ID Nr. 3 oder deren Gruppe B Analoge;
Seq ID Nr. 1 oder deren Gruppe B Analoge und Seq ID Nr. 5 oder deren Gruppe B Analoge;
Seq ID Nr. 3 oder deren Gruppe B Analoge und Seq ID Nr. 5 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge;
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 11 oder deren Gruppe B Analog und
Seq ID Nr. 1 oder deren Gruppe B Analoge und Seq ID Nr. 3 oder deren Gruppe B Analoge und Seq ID Nr. 5 oder deren Gruppe B Analoge und Seq ID Nr. 7 oder deren Gruppe B Analoge und Seq ID Nr. 11 oder deren Gruppe B Analoge,
darstellt.

2. *Pichia ciferrii* Zelle gemäß Anspruch 1, **dadurch gekennzeichnet, dass** die Verminderung der enzymatischen Aktivität erreicht wird durch Modifikation eines Genes umfassend eine der in Anspruch 1 genannten Nukleinsäuresequenzen, wobei die Modifikation ausgewählt ist aus der Gruppe umfassend Insertion von Fremd-DNA in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

3. *Pichia ciferrii* Zelle gemäß Anspruch 2, **dadurch gekennzeichnet, dass** die Fremd-DNA ein Selektionsmarkergen, bevorzugt eins, welches rückstandslos entfernt werden kann und eine Deletion im Ziel-Gen hinterlässt, ist.

4. *Pichia ciferrii* Zelle gemäß mindestens einem der vorherigen Ansprüche, **dadurch gekennzeichnet, dass** die *Pichia ciferrii* Zelle sich ableitet von Stämmen ausgewählt aus der Gruppe bestehend aus *Pichia ciferrii NRRL Y-1031 F-60-10, Pichia ciferrii* CS.PCΔPro2.

5. *Pichia ciferrii* Zelle gemäß mindestens einem der vorherigen Ansprüche, **dadurch gekennzeichnet, dass** die Zelle verglichen zu ihrem Wildtyp eine erhöhte enzymatische Aktivität mindestens eines der Enzyme ausgewählt aus
einem Enzym E₁, welches die Reaktion von Serin und Palmitoyl-CoA zu 3-Ketosphinganin katalysiert, insbesondere einer Serin-Palmitoyltransferase, insbesondere solche kodiert durch Seq ID Nr. 13 und /oder Seq ID Nr. 15, einem Enzym E₂, welches die Reaktion von Sphinganin zu Phytosphingosin katalysiert, insbesondere einer Sphinganin C4-Hydroxylase, insbesondere solcher kodiert durch Seq ID Nr. 17,

6. Verwendung der Zellen gemäß mindestens einem der vorherigen Ansprüche zur Herstellung von Sphingoidbasen und Sphingolipiden.

7. Verfahren zur Herstellung einer *Pichia ciferri* Zelle nach mindestens einem der Ansprüche 1 bis 5 umfassend die Verfahrensschritte:
I) Bereitstellen einer *Pichia ciferrii* Zelle und
II) Modifikation mindestens eines Genes umfassend eine der Sequenzen ausgewählt aus den in Anspruch 1 genannten Nukleinsäuresequenzgruppen A) und B) durch Insertion von Fremd-DNA, insbesondere von DNA kodierend für ein Selektionsmarkergen, in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

8. Verfahren zur Herstellung von Sphingoidbasen und Sphingolipiden umfassend die Verfahrensschritte
a) In Kontakt bringen der Zelle gemäß mindestens einem der Ansprüche 1 bis 5 mit einem Medium beinhaltend eine Kohlenstoffquelle,
b) Kultivieren der Zelle unter Bedingungen, die es der Zelle ermöglichen aus der Kohlenstoffquelle Sphingoidbasen und Sphingolipide zu bilden und
c) gegebenenfalls Isolierung der gebildeten Sphingoidbasen und Sphingolipide.

## Claims

1. *Pichia ciferrii* cell, **characterized in that** the cell has, compared to its wild type, a reduced activity of at least one of the enzymes which are encoded by the intron-free nucleic acid sequences selected from the two groups A) and B) consisting of
A) Seq ID No 1, Seq ID No 3, Seq ID No 5, Seq ID No 7, Seq ID No 9, Seq ID No 11,
B) a sequence which is at least 80% identical to any of the sequences Seq ID No 1, Seq ID No 3, Seq ID No 5, Seq ID No 7, Seq ID No 9, Seq ID No 11,
**characterized in that** the reduced activity of at least one of the enzymes represents a combination of reduced activities of the enzymes selected from the group:
Seq ID No 3 or its group B analogue;
Seq ID No 5 or its group B analogue;
Seq ID No 7 or its group B analogue;
Seq ID No 9 or its group B analogue;
Seq ID No 11 or its group B analogue;
Seq ID No 1 or its group B analogue and Seq ID No 3 or its group B analogue;
Seq ID No 1 or its group B analogue and Seq ID No 5 or its group B analogue;
Seq ID No 3 or its group B analogue and Seq ID No 5 or its group B analogue;
Seq ID No 1 or its group B analogue and Seq ID No 3 or its group B analogue and Seq ID No 5 or its group B analogue;
Seq ID No 1 or its group B analogue and Seq ID No 3 or its group B analogue and Seq ID No 5 or its group B analogue and Seq ID No 7 or its group B analogue;
Seq ID No 1 or its group B analogue and Seq ID No 3 or its group B analogue and Seq ID No 5 or its group B analogue and Seq ID No 11 or its group B analogue and
Seq ID No 1 or its group B analogue and Seq ID No 3 or its group B analogue and Seq ID No 5 or its group B analogue and Seq ID No 7 or its group B analogue and Seq ID No 11 or its group B analogue.

2. *Pichia ciferrii* cell according to Claim 1, **characterized in that** reduction of the enzymic activity is achieved by modifying a gene comprising any of the nucleic acid sequences specified in Claim 1, the modification being selected from the group comprising
insertion of foreign DNA into the gene, deletion of at least parts of the gene, point mutations in the gene sequence, exposing the gene to the influence of RNA interference, and replacement of parts of the gene with foreign DNA, in particular of the promoter region.

3. *Pichia ciferrii* cell according to Claim 2, **characterized in that** the foreign DNA is a selection marker gene, preferably one which can be removed without leaving a trace and which leaves a deletion in the target gene.

4. *Pichia ciferrii* cell according to at least one of the preceding claims, **characterized in that** the *Pichia ciferrii* cell derives from strains selected from the group consisting of *Pichia ciferrii NRRL Y-1031 F-60-10, Pichia ciferrii* CS.PCAPro2.

5. *Pichia ciferrii* cell according to at least one of the preceding claims, **characterized in that** the cell has, compared to its wild type, an increased enzymic activity of at least one of the enzymes selected from
an enzyme E₁, which catalyses the reaction of serine and palmitoyl-CoA to give 3-ketosphinganine, in particular a serine palmitoyl transferase, in particular those encoded by Seq ID No 13 and/or Seq ID No 15,
an enzyme E₂, which catalyses the reaction of sphinganine to phytosphingosine, in particular a sphinganine C4-hydroxylase, in particular that encoded by Seq ID No 17.

6. Use of the cells according to at least one of the preceding claims for producing sphingoid bases and sphingolipids.

7. Method of producing a *Pichia ciferri* cell according to at least one of Claims 1 to 5, said method comprising the steps of:
I) providing a *Pichia ciferrii* cell, and
II) modifying at least one gene comprising any of the sequences selected from the nucleic acid sequence groups A) and B) specified in Claim 1 by insertion of foreign DNA, in particular DNA coding for a selection marker gene, into the gene, deletion of at least parts of the gene, point mutations in the gene sequence, exposing the gene to the influence of RNA interference, and replacement of parts of the gene with foreign DNA, in particular of the promoter region.

8. Method of producing sphingoid bases and sphingolipids, said method comprising the steps of
a) contacting the cell according to at least one of Claims 1 to 5 with a medium including a carbon source,
b) culturing the cell under conditions which enable the cell to produce sphingoid bases and sphingolipids from said carbon source, and
c) optionally isolating the sphingoid bases and sphingolipids produced.

## Revendications

1. Cellule de *Pichia ciferrii,* **caractérisée en ce que** la cellule présente une activité réduite, par comparaison avec son type sauvage, d'au moins l'une des enzymes qui sont codées par les séquences d'acide nucléique sans introns choisies dans les deux groupes A) et B) consistant en
A) SEQ ID N° 1, SEQ ID N° 3, SEQ ID N° 5, SEQ ID N° 7, SEQ ID N° 9, SEQ ID N° 11,
B) une séquence qui est identique à raison d'au moins 80 % à l'une des séquences SEQ ID N° 1, SEQ ID N° 3, SEQ ID N° 5, SEQ ID N° 7, SEQ ID N° 9, SEQ ID N° 11,
**caractérisée en ce que** l'activité réduite d'au moins l'une des enzymes représente une combinaison d'activités réduites des enzymes choisies dans le groupe :
SEQ ID N° 3 ou ses analogues du groupe B ;
SEQ ID N° 5 ou ses analogues du groupe B ;
SEQ ID N° 7 ou ses analogues du groupe B ;
SEQ ID N° 9 ou ses analogues du groupe B ;
SEQ ID N° 11 ou ses analogues du groupe B ;
SEQ ID N° 1 ou ses analogues du groupe B et SEQ ID N° 3 ou ses analogues du groupe B ;
SEQ ID N° 1 ou ses analogues du groupe B et SEQ ID N° 5 ou ses analogues du groupe B ;
SEQ ID N° 3 ou ses analogues du groupe B et SEQ ID N° 5 ou ses analogues du groupe B ;
SEQ ID N° 1 ou ses analogues du groupe B et SEQ ID N° 3 ou ses analogues du groupe B et SEQ ID N° 5 ou ses analogues du groupe B ;
SEQ ID N° 1 ou ses analogues du groupe B et SEQ ID N° 3 ou ses analogues du groupe B et SEQ ID N° 5 ou ses analogues du groupe B et SEQ ID N° 7 ou ses analogues du groupe B ;
SEQ ID N° 1 ou ses analogues du groupe B et SEQ ID N° 3 ou ses analogues du groupe B et SEQ ID N° 5 ou ses analogues du groupe B et SEQ ID N° 11 ou ses analogues du groupe B et
SEQ ID N° 1 ou ses analogues du groupe B et SEQ ID N° 3 ou ses analogues du groupe B et SEQ ID N° 5 ou ses analogues du groupe B et SEQ ID N° 7 ou ses analogues du groupe B et SEQ ID N° 11 ou ses analogues du groupe B,

2. Cellule de *Pichia ciferrii* selon la revendication 1, **caractérisée en ce que** la diminution de l'activité enzymatique est atteinte par modification d'un gène comprenant une des séquences d'acide nucléique nommées dans la revendication 1, la modification étant choisie dans le groupe comprenant une insertion d'ADN étranger dans le gène, une délétion au moins de parties du gène, des mutations ponctuelles dans la séquence du gène, une exposition du gène à l'effet de l'interférence par ARN et un remplacement de parties du gène par de l'ADN étranger, en particulier de la région de promoteur.

3. Cellule de *Pichia ciferrii* selon la revendication 2, **caractérisée en ce que** l'ADN étranger est un gène marqueur de sélection, de préférence un qui peut être éliminé sans résidu et laisse une délétion dans le gène cible.

4. Cellule de *Pichia ciferrii* selon au moins l'une quelconque des revendications précédentes, **caractérisée en ce que** la cellule de *Pichia ciferrii* dérive de souches choisies dans le groupe constitué par *Pichia ciferrii NRRL Y-1031 F-60-10, Pichia ciferrii* CS.PCΔPro2.

5. Cellule de *Pichia ciferrii* selon au moins l'une quelconque des revendications précédentes, **caractérisée en ce que** la cellule, comparée à son type sauvage, présente une activité enzymatique accrue d'au moins l'une des enzymes choisies parmi
une enzyme E₁ qui catalyse la conversion de sérine et palmitoyl-CoA en 3-cétosphinganine, en particulier d'une sérine-palmitoyltransférase, en particulier celle(s) codée(s) par SEQ ID N° 13 et/ou SEQ ID N° 15,
une enzyme E₂ qui catalyse la conversion de sphinganine en phytosphingosine, en particulier d'une sphinganine C4-hydrolase, en particulier de celle codée par SEQ ID N° 17.

6. Utilisation de cellules selon au moins l'une quelconque des revendications précédentes, pour la production de bases sphingoïdes et de sphingolipides.

7. Procédé pour la production d'une cellule de *Pichia ciferrii* selon au moins l'une quelconque des revendications 1 à 5, comprenant les étapes de processus :
I) disposition d'une cellule de *Pichia ciferrii* et
II) modification d'au moins in gène comprenant l'une des séquences choisies dans les groupes de séquences d'acide nucléique A) et B) nommées dans la revendication 1, par insertion dans le gène d'ADN étranger, en particulier d'ADN codant pour un gène marqueur de sélection, délétion au moins de parties du gène, mutations ponctuelles dans la séquence du gène, exposition du gène à l'effet de l'interférence par ARN et remplacement de parties du gène par de l'ADN étranger, en particulier de la région de promoteur.

8. Procédé pour la production de bases sphingoïdes et de sphingolipides, comprenant les étapes de processus
a) mise en contact de la cellule selon au moins l'une quelconque des revendications 1 à 5, avec un milieu comportant une source de carbone,
b) culture de la cellule dans des conditions qui permettent à la cellule de former des bases sphingoïdes et des sphingolipides à partir de la source de carbone et
c) éventuellement isolement des bases sphingoïdes et des sphingolipides.
